(19) Europäisches Patentamt / European Patent Office / Office européen des brevets

(11) **EP 1 549 599 B1**

(12) **EUROPÄISCHE PATENTSCHRIFT**

(45) Veröffentlichungstag und Bekanntmachung des Hinweises auf die Patenterteilung:
**08.07.2009 Patentblatt 2009/28**

(21) Anmeldenummer: **03775161.7**

(22) Anmeldetag: **18.09.2003**

(51) Int Cl.:
***C07C 43/23*** *(2006.01)* ***C07C 39/15*** *(2006.01)*
***C07C 39/367*** *(2006.01)* ***C07C 39/06*** *(2006.01)*
***C09K 19/58*** *(2006.01)*

(86) Internationale Anmeldenummer:
**PCT/EP2003/010398**

(87) Internationale Veröffentlichungsnummer:
**WO 2004/033406 (22.04.2004 Gazette 2004/17)**

(54) **CHIRALES PHENOLDERIVAT, DIESES ENTHALTENDES FLÜSSIGKRISTALLMEDIUM**

CHIRAL PHENOL DERIVATIVE, LIQUID CRYSTAL MEDIUM CONTAINING SAID CHIRAL PHENOL DERIVATIVE

DERIVE DE PHENOL CHIRAL, SUPPORT DE CRISTAUX LIQUIDES LE CONTENANT

(84) Benannte Vertragsstaaten:
**AT BE BG CH CY CZ DE DK EE ES FI FR GB GR HU IE IT LI LU MC NL PT RO SE SI SK TR**

(30) Priorität: **07.10.2002 DE 10246657**

(43) Veröffentlichungstag der Anmeldung:
**06.07.2005 Patentblatt 2005/27**

(73) Patentinhaber: **Merck Patent GmbH**
**64293 Darmstadt (DE)**

(72) Erfinder:
- **REIFFENRATH, Volker**
**64380 Rossdorf (DE)**
- **HECKMEIER, Michael**
**69502 Hemsbach (DE)**

(56) Entgegenhaltungen:
**EP-A- 0 217 239** **DE-A- 10 117 224**
**DE-A- 19 539 141**

- **MAEURER, M. ET AL: "Stereoelectronic and steric effects in the synthesis and recognition of diastereomeric ethers by NMR and EPR spectroscopy" CHEMISCHE BERICHTE , 125(4), 857-65 CODEN: CHBEAM; ISSN: 0009-2940, 1992, XP002272381**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft chirale Phenolderivate, sowie flüssigkristalline Medien, die diese Verbindungen enthalten. Die vorliegende Erfindung betrifft auch ein Verfahren zur chiralen Dotierung und gleichzeitigen Stabilisierung von Flüssigkristallmischungen. Ferner betrifft die vorliegende Erfindung Flüssigkristallanzeigen, die die erfindungsgemäßen Flüssigkristallmischungen enthalten.

In den bekannten Flüssigkristallanzeigen werden die Flüssigkristalle, in der Regel flüssigkristalline Gemische, als Dielektrika verwendet, deren optische Eigenschaften sich bei Anlegen einer elektrischen Spannung reversibel ändern. Diese Flüssigkristallanzeigen verwenden verschiedene elektrooptische Effekte. Die gebräuchlichsten hiervon sind der TN-Effekt (Englisch "twisted nematic"), mit einer homogenen, nahezu planaren Ausgangsorientierung der Flüssigkristalle und einer um ca. 90° verdrillten nematischen Struktur), der STN-Effekt (Englisch "supertwisted nematic") und der SBE-Effekt (Englisch "supertwisted birefringence effect"), die beide, wie auch der TN-Effekt, eine verdrillte, homogene Ausgangsorientierung der Flüssigkristalle verwendet, wobei hier jedoch die Moleküle einen signifikanten Anstellwinkel an der Oberfläche der Substrate (Englisch "surface tilt angle" kurz "tilt angle") haben und die Verdrillung (Englisch "twist") zwischen den Substraten deutlich größer als 90° ist. In dieser Anmeldung werden im folgenden, wenn nicht explizit anders angegeben, der STN-Effekt und der SBE-Effekt beide gemeinsam als STN-Effekt bezeichnet. Der Anstellwinkel an der Oberfläche beträgt bei den STN-Anzeigen typischer Weise zwischen 2° und 10°. Er ist um so höher, je größer der Verdrillungswinkel ist. Die Verdrillungswinkel betragen in der Regel ca. 180° bis 240°, manchmal auch bis 260° oder 270° und in einigen Fällen sogar noch mehr.

Die Verdrillung des Flüssigkristallmediums von mehr als 90° wird durch den Einsatz von chiralen Flüssigkristallmischungen erreicht, deren natürliche Verdrillung entsprechend der Schichtdicke der Flüssigkristallschicht gewählt wird. Hierzu stehen dem Fachmann zwei Möglichkeiten zur Verfügung. Die erste beseht in der Verwendung von Flüssigkristallen, die selbst chiral sind, also von cholesterischen Flüssigkristallen. Solche Flüssigkristalle besitzen selbst eine verdrillte Struktur. In einer homogen orientierten Anordnung zwischen zwei Substraten, die Grand-Jean Textur genannt wird, ist der Direktor der Moleküle in vertikaler Richtung, also über die Dicke der Schicht, schraubenförmig verdrillt.

Die charakteristische Länge für eine komplette Drehung um 360° wird der cholesterische Pitch (P) genannt. Die Verwendung cholesterischer Flüssigkristalle ist jedoch oft nicht besonders vorteilhaft, da der cholesterische Pitch cholesterischer Flüssigkristalle sich nicht einfach an die Schichtdicken der üblicherweise verwendeten Zellen der Anzeigen anpassen läßt. Zusätzlich hängt der cholesterische Pitch dieser Flüssigkristalle oft in unvorteilhafter Weise und in vielen Fällen stark von der Temperatur ab. Auch führt eine Veränderung der Zusammensetzung der Mischungen meist zu starken Änderungen des cholesterischen Pitches.

Aus diesem Grunde wird in den meisten praktischen Fällen eine nematische Flüssigkristallmischung mit einer chiralen Substanz versetzt, die die gewünschte Verdrillung induziert. Hierbei ist es nicht besonders wesentlich, ob diese Verbindung selbst eine Mesophase aufweist. Wichtiger ist vielmehr, dass sie ein hohes Verdrillungsvermögen für die nematische Basismischung (auch Wirtsmischung, Englisch "host mixture" genannt) besitzt und dass sie in den üblicherweise eingesetzten Konzentrationen die Eigenschaften der Basismischung, insbesondere deren Klärpunkt, nicht zu stark verändert. Somit werden in der Regel bevorzugt Verbindungen eingesetzt, die selbst eine mesogene Struktur aufweisen oder sogar cholesterisch sind.

Die cholesterischen Phasen, die durch Zugabe chiraler Substanzen zu nematischen Flüssigkristallen induzierten werden, werden oft als chiral nematische Phasen bezeichnet. In der vorliegenden Anmeldung werden jedoch auch diese als cholesterische Phasen bezeichnet, wenn nicht explizit anders angegeben.

Der cholesterische Pitch, der durch Zugabe chiraler Substanzen (Dotierstoffe) zu nematischen Flüssigkristallen induziert wird, hängt bei gegebener Temperatur, neben der Enantiomerenreinheit des chiralen Dotierstoffs, insbesondere von der eingesetzten Konzentration des Dotierstoffs (c) und von dessen Verdrillungsvermögen ab. Dieses Verdrillungsvermögen wird HTP (von Englisch "helical twisting power") genannt. In erster Näherung ist der induzierte cholesterische Pitch (P) umgekehrt proportional zum Produkt aus HTP und eingesetzter Konzentration des Dotierstoffs, wie in Gleichung (1) gezeigt.

$$P = (HTP \cdot c)^{-1} \qquad (1)$$

Bei STN-Anzeigen werden typischerweise Flüssigkristallmischungen mit einem Verhältnis des cholestrischen Pitchs zur Schichtdicke (d/P) im Bereich von 0,4 bis 0,8, oft von ca. 0,5 verwendet.

Aber auch in TN-Anzeigen werden chirale Flüssigkristallmischungen verwendet, hier zur Vermeidung der Verdrillung mit dem umgekehrten Drehsinn (Englisch "reverse twist"). Deren Auftreten würde zur Bildung von Domänen und damit zu einer Verringerung des Kontrasts führen. Bei TN-Anzeigen werden in der Regel cholesterische Flüssigkristallmischungen mit einem deutlich kleineren d/P-Verhältnis verwendet, als in STN-Anzeigen, da größere d/P-Werte in den meisten Fällen zu einer Erhöhung der Schwellenspannung führen. Typischer Weise betragen die Werte hier ca. 0,01 bis 0,3, oft ca. 0,1.

Neben diesen Anzeigetypen gibt es weitere Flüssigkristallanzeigen, die mit chiralen Verbindungen dotierte Flüssigkristallmischungen verwenden.

Als chirale Dotierstoffe sind z.B. die Verbindungen C15, CB15, R-811 und S-811, R-1011 und S-1011, sowie R-2011 und S-2011, alle Merck KGaA bekannt.

Bei diesen und ähnlichen elektrooptischen Effekten werden flüssigkristalline Medien mit positiver dielektrischer Anisotropie ($\Delta\varepsilon$) verwendet.

Neben den genannten elektrooptischen Effekten, welche Flüssigkristallmedien mit positiver dielektrischer Anisotropie benötigen, gibt es andere elektrooptische Effekte welche Flüssigkristallmedien mit negativer dielektrischer Anisotropie verwenden, wie z.B. der ECB-Effekt ("Electrically Controlled Birefringence") und seine Unterformen DAP ("Deformation of Aligned Phases"), VAN ("Vertically Aligned Nematics") und CSH ("Colour Super Homeotropics"). Bei diesen und ähnlichen elektrooptischen Effekten werden flüssigkristalline Medien mit negativer dielektrischer Anisotropie ($\Delta\varepsilon$) verwendet.

Ein elektrooptischer Effekt mit hervorragender, kleiner Blickwinkelabhängigkeit des Kontrasts verwendet axial symmetrische Micropixel (ASM von Englisch "Axially Symmetric Micro Pixel"). Bei diesem Effekt ist der Flüssigkristall jedes Pixels zylinderförmig von einem Polymermaterial umgeben. Dieser Mode eignet sich besonders zur Kombination mit der Adressierung durch Plasmakanäle. So lassen sich insbesondere großflächige PA LCDs mit guter Blickwinkelabhängigkeit des Kontrasts realisieren.

Der in letzter Zeit verstärkt eingesetzte IPS-Effekt ("In Plane Switching") kann sowohl dielektrisch positive wie auch dielektrisch negative Flüssigkristallmedien verwenden, ähnlich wie auch "guest host"-Anzeigen also Gast/Wirt-Anzeigen, die Farbstoffe je nach verwandtem Anzeigemodus entweder in dielektrisch positiven oder in dielektrisch negativen Medien einsetzen können.

Die Bildpunkte der Flüssigkristallanzeigen können direkt angesteuert werden, zeitsequentiell, also im Zeitmultiplexverfahren oder mittels einer Matrix von aktiven, elektrisch nichtlinearen Elementen angesteuert werden.

Die bislang gebräuchlichsten AMDs (Englisch "active matrix displays") verwenden diskrete aktive elektronische Schaltelemente, wie z. B. dreipolige Schaltelemente wie MOS (Englisch "metal oxide silicon") Transistoren oder Dünnfilmtransistoren (TFTs von Englisch "thin film transistors") oder Varistoren oder 2-polige Schaltelemente wie z.B. MIMs (Englisch "metall insulator metal") Dioden, Ringdioden oder "back to back"-Dioden. Bei den TFTs werden verschiedene Halbleitermaterialien, überwiegend Silizium oder auch Cadmiumselenid, verwendet. Insbesondere wird amorphes Silizium oder polykristallines Silizium verwendet.

Einige flüssigkristalline Verbindungen und einige mesogene Verbindungen mit ansonsten anwendungstechnisch günstigen Eigenschaften sind nicht ausreichend stabil für den Einsatz in praktischen Flüssigkristallanzeigen. Für die unzureichende Stabilität der verschiedenen Verbindungen gibt es unterschiedliche Gründe. Diese können in ungenügender Stabilität der Verbindungen gegen UV-Strahlung und/oder sichtbares Licht oder in ihrer ungenügenden Stabilität gegen thermische Belastung liegen.

Einige Verbindungen reagieren bereits mit Spuren von Sauerstoff. Dies geschieht beschleunigt bei höheren Temperaturen. Bei Flüssigkristallmischungen, die derartige Verbindungen enthalten, läßt sich die Stabilität gegenüber thermischer Belastung, und damit die Lebensdauer der Anzeigen, deutlich erhöhen, indem entsprechende Stabilisatoren zugesetzt werden.

So wird in DE 195 391 41 und DE 101 172 24 ein Phenol der Formel

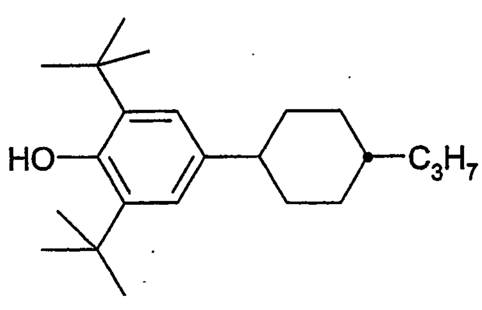

als Stabilisator für derartige Flüssigkristallmischungen vorgeschlagen. Diese Verbindungen sind nicht chiral und sind somit nicht geeignet in nematischen Flüssigkristallen eine cholesterische Phase zu induzieren. Mäurer, M. et. al. Chem. Ber. (1992), 125(4), S. 857 - 865 offenbart diastereomere, also achirale Phenolverbindungen. EP 0 217 239 offenbart

chirale Phenolester mesogener Carbonsäuren und deren Verwendung als Dotierstoff in Flüssigkristall-Phasen.

Sollen Flüssigkristallmischungen, die Verbindungen mit relativ geringer Stabilität gegen thermische Belastung in Gegenwart von Sauerstoff in Anzeigen verwendet werden, die cholesterische Flüssigkristalle benötigen, müssen die nematischen Basismischungen sowohl mit einem chiralen Dotierstoff, als auch mit einem Stabilisator versetzt werden. Dies bedeutet mindestens zwei zusätzliche Schritte in der Produktion derartiger Mischungen.

Somit besteht der Bedarf nach Verbindungen, die es erlauben den gewünschten cholesterischen Pitch in nematischen Basismischungen zu induzieren und diese Mischungen gleichzeitig zu stabilisieren.

Es wurde gefunden, dass diese Anforderung durch Verwendung entsprechender Verbindungen gelöst werden kann.

Als geeignet haben sich chirale Verbindungen der Formel I

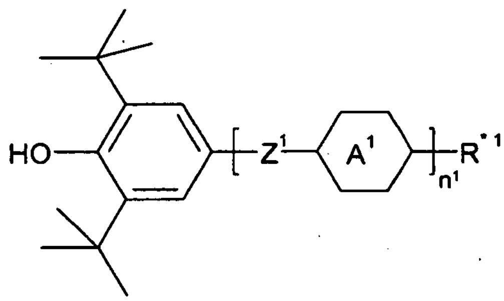

worin

$R^{*1}$ einen chiralen Rest der Formel I*, mit I*, K, L, M und N wie in Anspruch 1 definiert,

$Z^1$, wenn mehrfach vorhanden jeweils unabhängig voneinander, $-CH_2-CH_2-$, -CH=CH-, -C≡C-, -COO-, -OCO-, $-CH_2O-$, $-OCH_2-$, $-CF_2O-$, $-OCF_2-$, $-(CH_2)_4-$,- CF=CF-, -CH=CF-, -CF=CH-, $-CH_2-$, $-CF_2-$, -CF-, -O-, -S- oder eine Einfachbindung,

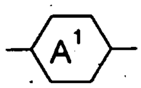,

wenn mehrfach vorhanden, jeweils unabhängig voneinander,

(a) einen trans-1,4-Cyclohexylenrest, worin auch eine oder mehrere nicht benachbarte $CH_2$-Gruppen durch -O- und/oder -S- ersetzt sein können,
(b) einen 1,4-Cyclohexenylenrest,
(c) einen 1,4-Phenylenrest, worin auch eine oder zwei CH-Gruppen durch N ersetzt sein können oder
(d) einen Rest ausgewählt aus der Gruppe
1,4-Bicyclo-(2,2,2)-octylen,
Piperidin-1,4-diyl, Naphtalin-2,6-diyl,
Decahydronaphthalin-2,6-diyl und
1,2,3,4-Tetrahydronaphthalin-2,6-diyl,

wobei diese Reste (a) bis (d) und der phenolische Benzolring optional einfach oder mehrfach durch F-Atome substituiert sein können, und

$n^1$ 0, 1, 2 oder 3

bedeuten,

**dadurch gekennzeichnet, dass** sie in einem nematischen Flüssigkristall eine cholesterische Phase induzieren und gleichzeitig als Stabilisator wirken,

erwiesen.

Bevorzugt bedeuten in Formel I

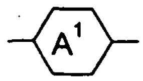

wenn mehrfach vorhanden, jeweils unabhängig voneinander,

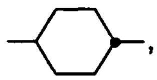

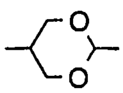, 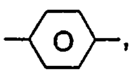, 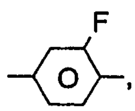, 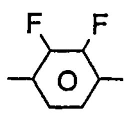 oder 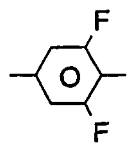

$Z^1$, wenn mehrfach vorhanden, jeweils unabhängig voneinander, $-CH_2-CH_2-$, -CH=CH-, -C≡C-, -COO-oder eine Einfachbindung, besonders bevorzugt $-CH_2-CH_2-$ oder eine Einfachbindung, insbesondere bevorzugt eine Einfachbindung und

$n^1$ 0, 1 oder 2, bevorzugt 0 oder 1.

Bevorzugt sind ferner Verbindungen der Formel Ia

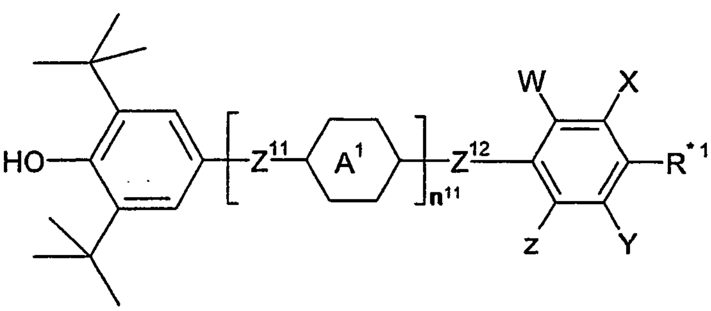

Ia

worin

$R^{*1}$ und

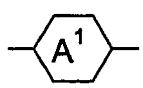

die oben bei Formel I gegebene Bedeutung und

$Z^{11}$ und $Z^{12}$, jeweils unabhängig voneinander, die oben bei Formel I für $Z^1$ gegebene Bedeutung

haben, und

$n^{11}$ 0, 1 oder 2, bevorzugt 0 oder 1 und

W, X, Y und Z, jeweils unabhängig voneinander, H, F, Cl, Alkyl, oder Alkoxy, bevorzugt mit 1 bis 7 C-Atomen

bedeuten.

Des weiteren sind bevorzugt Verbindungen der Formel I, bevorzugt der Formel Ia, **dadurch gekennzeichnet, dass**

$R^{*1}$ einen chiralen Rest der Formel

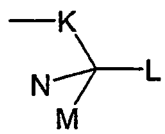

bedeutet, worin

K eine Einfachbindung, Alkylen mit 1 bis 5 C-Atomen, Alkenylen oder Alkinylen mit 2 bis 9, bevorzugt mit 2 bis 5 C-Atomen, wobei bei allen drei Arten von Gruppen jeweils eine, zwei oder mehr der vorhandenen $-CH_2-$ Gruppen durch -O-, -C=O- oder -S- ersetzt sein können, wobei jedoch keine zwei O-Atome direkt miteinander verbunden sind und alle drei Arten von Gruppen optional durch Halogen, bevorzugt durch Fluor substituiert sein können, bevorzugt eine Einfachbindung, $-CH_2-$, -O-, -CO-O-, $-CO-O-CH_2-$, -O-CO-, $-CH_2-CH_2-$, -CH=CH- oder -C≡C- und

L, M und N, jeweils unabhängig voneinander, aber verschieden voneinander und vom Rest des Moleküls inklusive der Gruppe K, Wasserstoff, Halogen, bevorzugt F, Aryl oder Cycloalkyl, Alkyl oder Alkoxy mit 1 bis 7 C-Atomen, Alkenyl, Alkenyloxy, Alkinyl oder Alkinyloxy mit 2 bis 7 C-Atomen, wobei bei allen sechs Arten von Gruppen jeweils eine, zwei oder mehr der vorhandenen $-CH_2-$Gruppen durch -O-, -C=O- oder -S-ersetzt sein können, wobei jedoch keine zwei O-Atome direkt miteinander verbunden sind und alle sechs Arten von Gruppen optional durch Halogen, bevorzugt durch Fluor substituiert sein können, bevorzugt Phenyl, Alkyl, Alkoxy, Alkenyl oder Alkinyl,

bedeuten.

Insbesondere bedeutet bei diesen Verbindungen

$R^{*1}$ einen chiralen Rest ausgewählt aus der Gruppe der Reste der Formeln

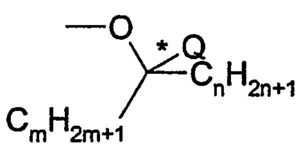 ,

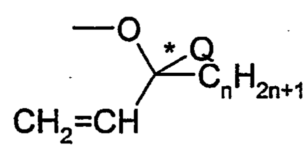 ,

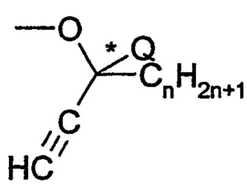 ,

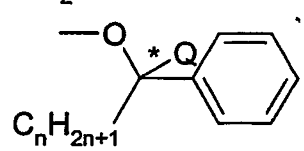 ,

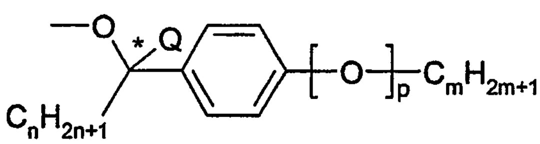 ,

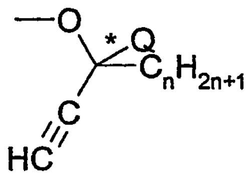
,
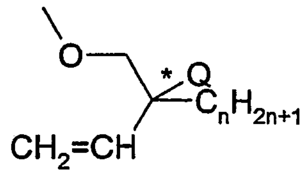
,

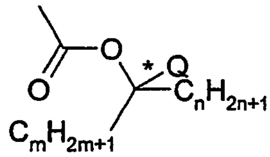
und
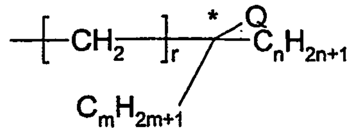

worin

Q H oder Halogen, bevorzugt H oder F, bevorzugt H,

n und m voneinander verschieden sind und ansonsten voneinander unabhängig 1 bis 11,

p 0 oder 1 und

r 0 bis 4, bevorzugt 0 bis 2

bedeuten.

Bevorzugt bedeutet

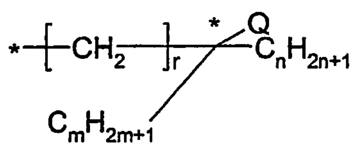

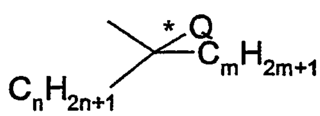
,
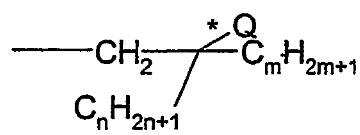

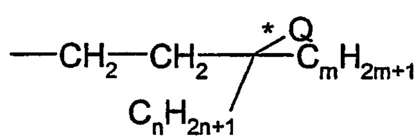
.

oder

worin die Parameter die oben gegebene Bedeutung haben.

Besonders bevorzugt sind Verbindung der Formel Ia, ausgewählt aus der Gruppe der Verbindungen der Formeln Ia-1 bis Ia-9

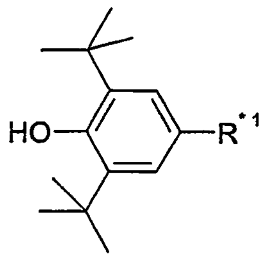

Ia-1

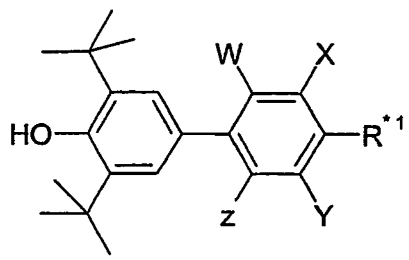

Ia-2

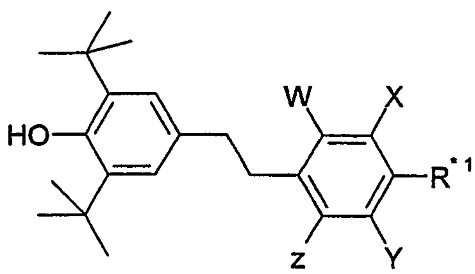

Ia-3

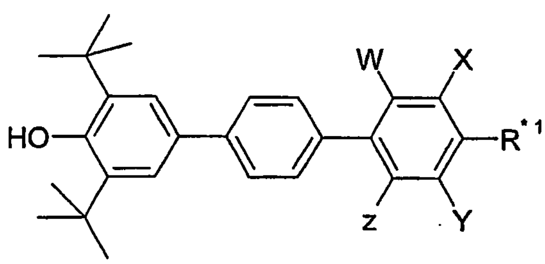

Ia-4

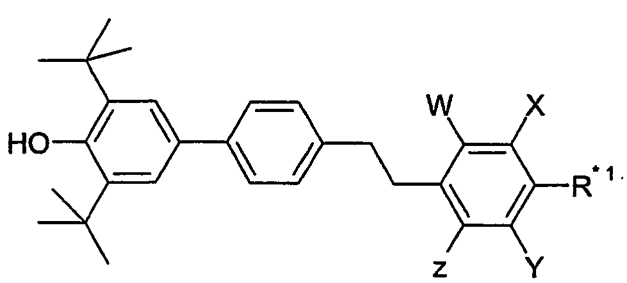

Ia-5

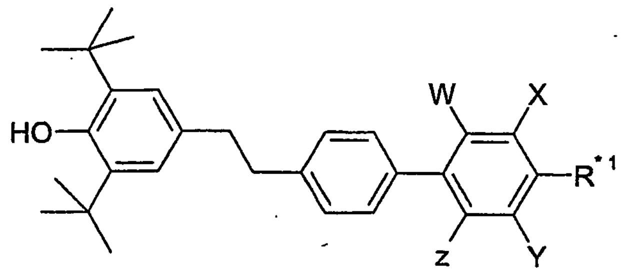

Ia-6

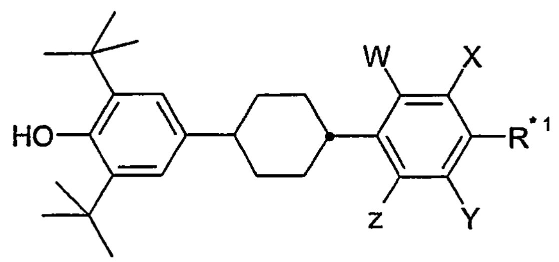

Ia-7

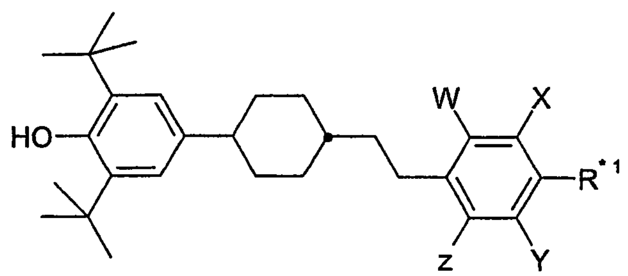

Ia-8

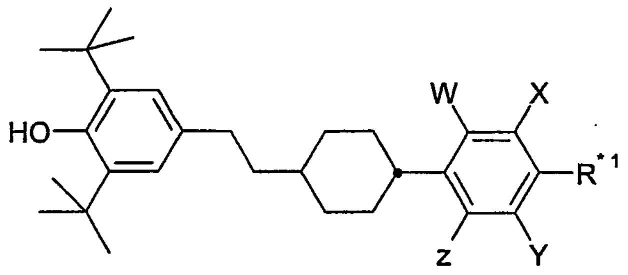

Ia-9

worin die Parameter die oben gegebene Bedeutung haben und

W und Z bevorzugt H bedeuten.

Die Verbindungen der Formel 1 werden gemäß Schema I und II hergestellt.

## Schema I

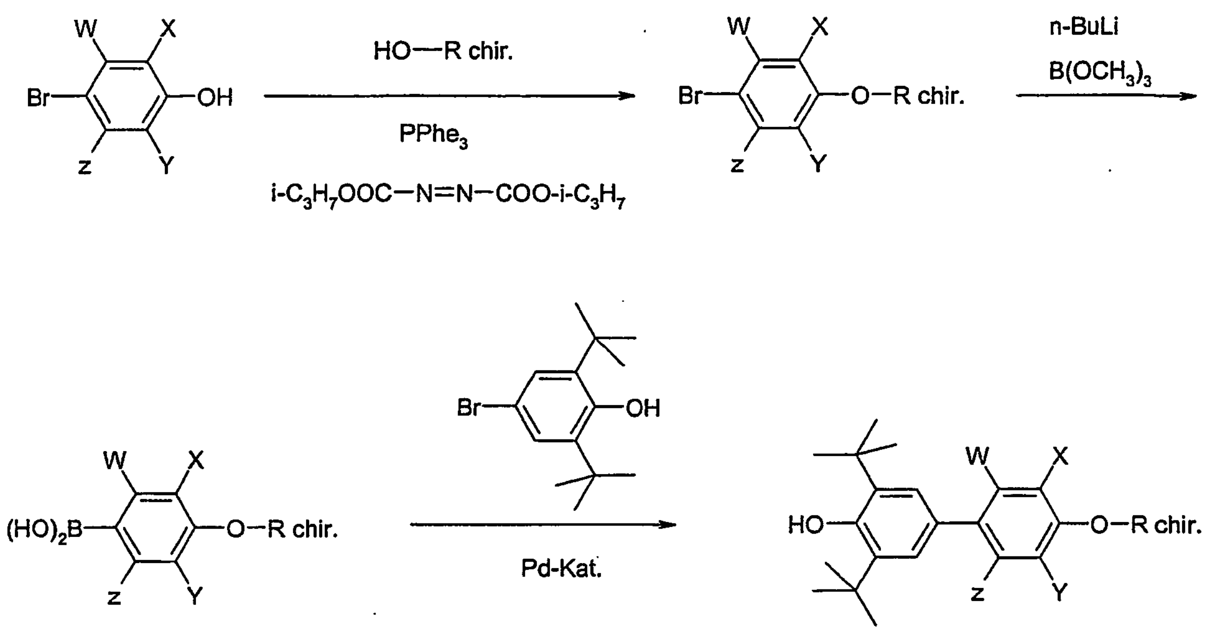


worin "R chir." $R^{*1}$ bedeutet und die Parameter die oben unter Formel I gegebene Bedeutung haben.

## Schema II

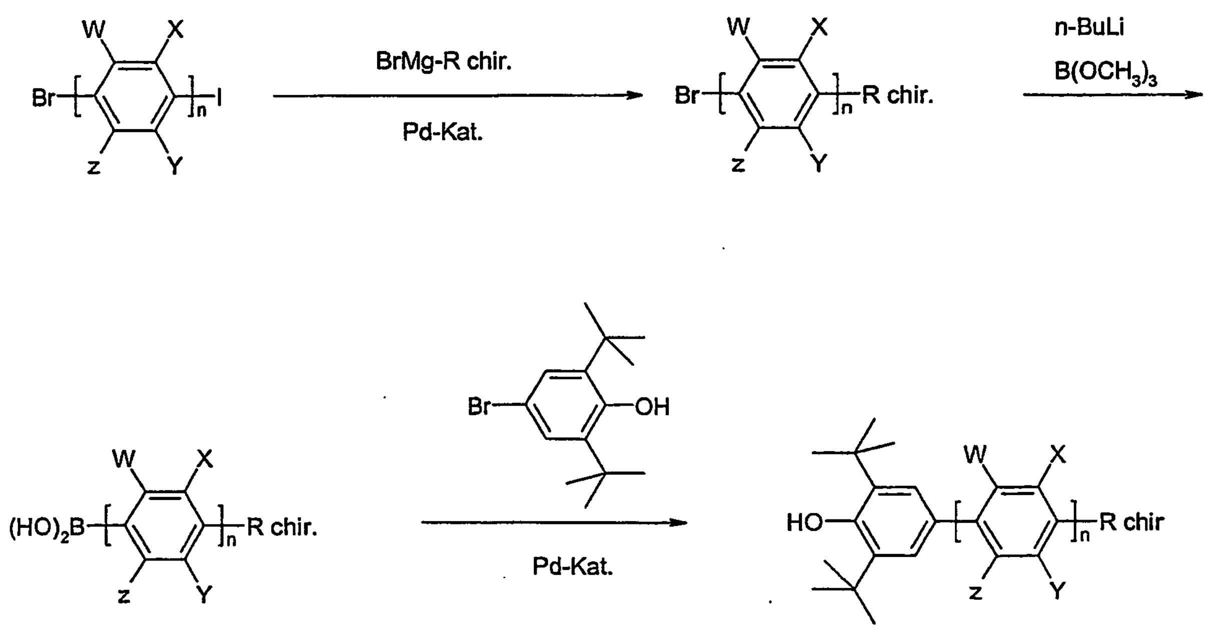


worin "R chir." $R^{*1}$ bedeutet und die Parameter die oben unter Formel I gegebene Bedeutung haben.

Die erfindungsgemäßen Flüssigkristallmedien enthalten eine oder mehrere Verbindungen der Formel I.

In einer bevorzugten Ausführungsform enthalten die Flüssigkristallmedien gemäß der vorliegenden Erfindung

a) eine oder mehrere chirale Verbindungen der Formel I

b) eine oder mehrere dielektrisch neutrale Verbindungen der Formel II

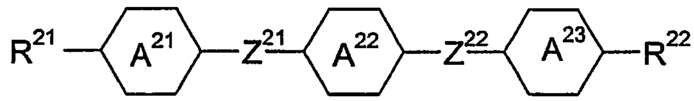

II

worin

$R^{21}$ und $R^{22}$ jeweils unabhängig voneinander, H, eine einfach durch CN oder $CF_3$ oder mindestens einfach durch Halogen substituierte Alkylgruppe mit 1 bis 15 C-Atomen, wobei auch eine oder mehrere $CH_2$-Gruppen, jeweils unabhängig voneinander, durch -O-, -S-, -CH=CH-, -CF=CF-, -CF=CH-, -CH=CF-,

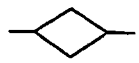,

, -CO-, -CO-O-, -O-CO- oder -O-CO-O- so ersetzt sein können, daß O-Atome nicht direkt miteinander verknüpft sind, bevorzugt Alkyl und Alkoxy mit 1 bis 12 C-Atomen, Alkoxyalkyl, Alkenyl oder Alkenyloxy mit 2 bis 12 C-Atomen,

$Z^{21}$ und $Z^{22}$ jeweils unabhängig voneinander die oben bei Formel I für $Z^{11}$ gegebene Bedeutung haben,

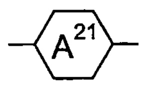

und

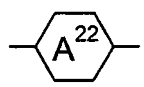

jeweils unabhängig voneinander

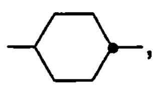,

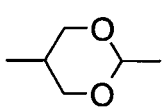, 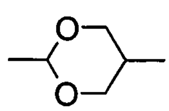, 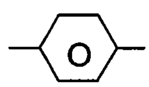,

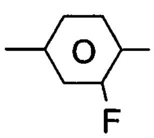, 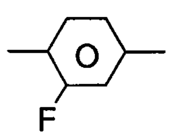 oder 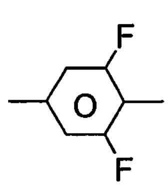

insbesondere bevorzugt bedeuten

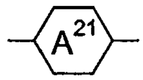 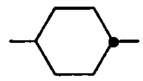

und

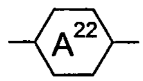 ,

wenn vorhanden,

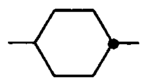 oder 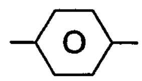 ,

l
0 oder 1
bedeuten,
und

c) eine oder mehrere dielektrisch positive Verbindungen oder

d) eine oder mehrere dielektrisch negative Verbindungen
und optional

e) eine oder mehrere dielektrisch neutrale Verbindung(en).

Die Flüssigkristallmedien gemäß der vorliegenden Erfindung enthalten bevorzugt eine oder mehrere Verbindungen, die mindestens eine olefinische Doppelbindung aufweisen und/oder die mindestens eine -CF2-O-Gruppe enthalten. Bevorzugt sind die Verbindungen, die mindestens eine olefinische Doppelbindung aufweisen, dielektrisch positiv oder dielktrisch neutral.

Besonders bevorzugt sind dielektrisch neutrale Verbindungen der Formel II, bei denen bevorzugt mindestens eine der folgenden Bedingungen erfüllt ist

a) $R^{21}$ ist Alkenyl,

b) $R^{22}$ ist Alkenyl und

c) mindestens eine der vorhandenen Brücken $Z^{21}$ und $Z^{22}$ ist -CH=CH-.

Bevorzugt enthalten die Flüssigkristallmedien eine oder mehrere Verbindungen der Formel II die keine Biphenyleinheit enthalten.

Besonders bevorzugt enthalten die Flüssigkristallmedien eine oder mehrere Verbindungen der Formel II worin zwei benachbarte Ringe direkt
verknüpft sind und zwar bevorzugt

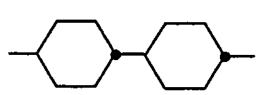

oder

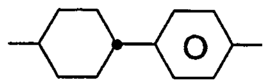

bedeuten.

Bevorzugt enthält das Flüssigkristallmedium eine oder mehrere Verbindungen ausgewählt aus der Gruppe der Verbindungen der Formeln II1 bis II4,

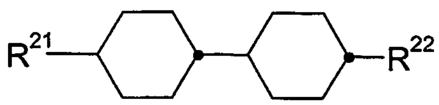

II1

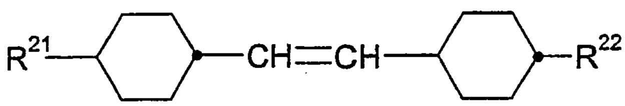

II2

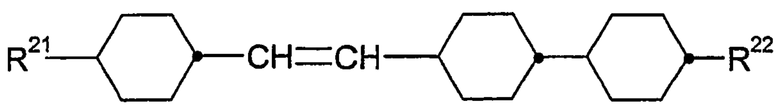

II3

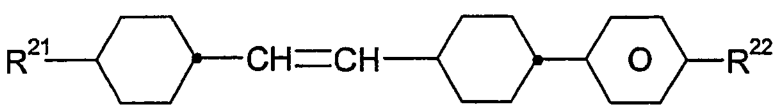

II4

worin

$R^{21}$ und $R^{22}$ jeweils die oben bei Formel II gegebene Bedeutung besitzen und in Formel II1 bevorzugt mindestens eine der Gruppen $R^{21}$ und $R^{22}$.Alkenyl bedeuten. Bevorzugt ist $R^{21}$ Alkyl oder Alkoxy mit 1-5 C-Atomen und $R^{22}$ Alkenyl.

Hier, wie in der gesamten vorliegenden Anmeldung, bedeutet, wenn nicht ausdrücklich anders angegeben, der Begriff Verbindungen, zur Verdeutlichung auch als Verbindung(en) geschrieben, sowohl eine Verbindung, als auch mehrere Verbindungen.

Bevorzugt enthält das Flüssigkristallmedium eine oder mehrere Verbindungen der Formel III

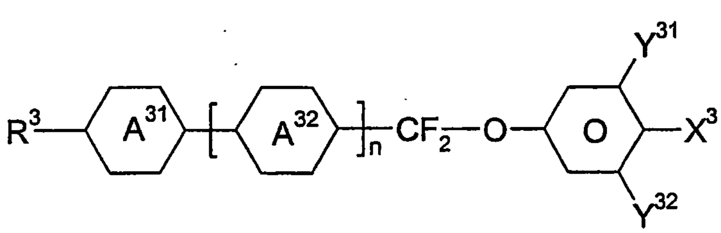

III

worin

$R^3$ Alkyl oder Alkoxy mit 1 bis 7 C-Atomen, Alkenyl, Alkenyloxy oder Oxaalkyl mit 2 bis 7 C-Atomen, bevortzugt Alkyl

oder Alkenyl, bevorzugt mit 1 bis 5, bzw. 2 bis 5 C-Atomen,

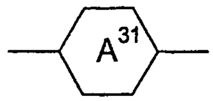 und 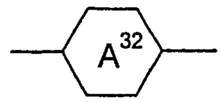 ,

jeweils unabhängig voneinander,

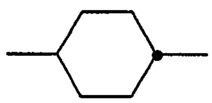 , 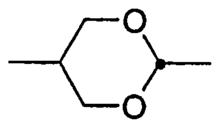 , 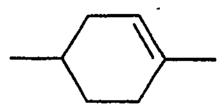 ,

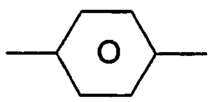 , 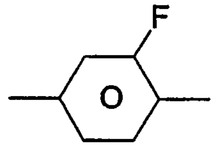 oder 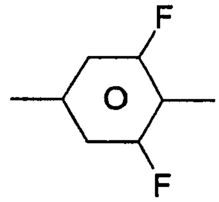 ,

bevorzugt

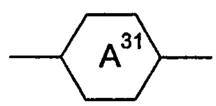

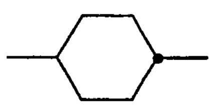 , 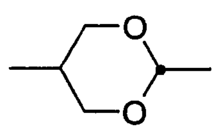 , 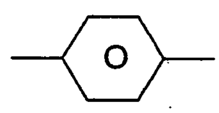

oder

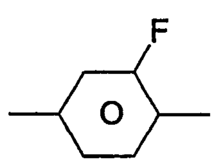

und

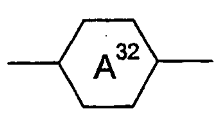

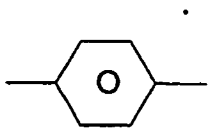 , 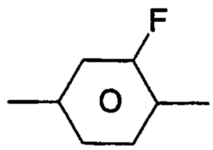 oder 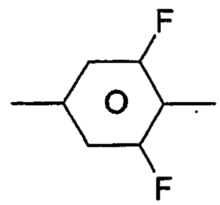 ,

$X^3$ F, Cl, $-OCF_2H$, $-OCF_3$ oder $-CF_3$, bevorzugt F, Cl oder $-CF_3$ und

$Y^{31}$ und $Y^{32}$, jeweils unabhängig voneoinander, H oder F bedeuten

bedeuten.

Bevorzugt enthält das Flüssigkristallmedium eine oder mehrere Verbindungen der Formel III ausgewählt aus der Gruppe der Verbindungen der Formeln III1 bis III5, bevorzugt III1, III4 und III5, besonders bevorzugt III1 und III5,

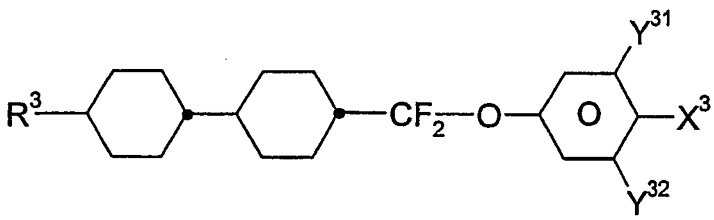 III1

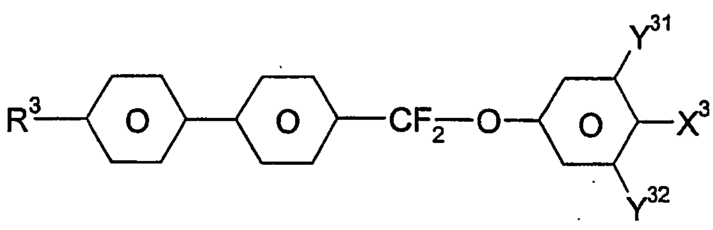 III2

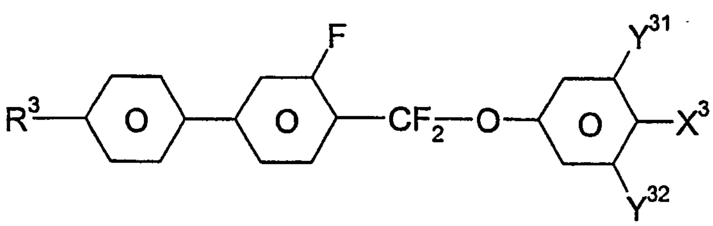 III3

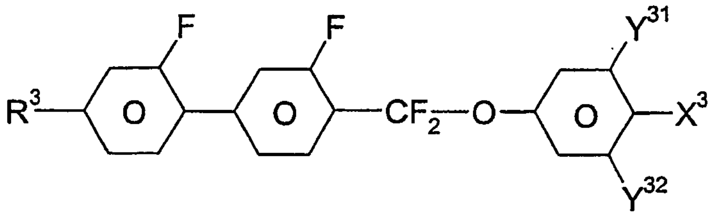

III4

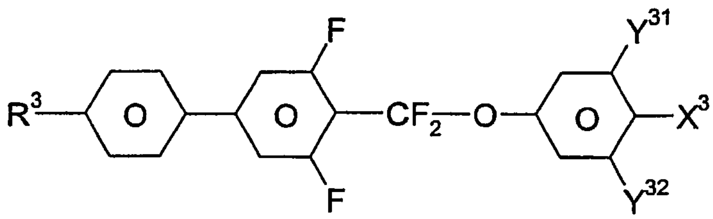

III5

worin die Parameter die oben unteer Formel III gegebene Bedeutung haben und bevorzugt

| | |
|---|---|
| $R^3$ | Alkyl oder Alkennyl und |
| $X^3$ und $Y^{31}$ | beide F und $Y^{32}$ H oder F, bevorzugt F oder |
| $X^3$ | $-OCF_3$ und $Y^{31}$ F und $Y^{32}$ H oder F, bevorzugt H |

bedeuten.

Bei den nematischen oder nematogenen Verbindungen, insbesondere bei den Verbindungen der Formeln II und III werden, werden in der Regel, die einzelnen Verbindungen in Konzentrationen von 1 % bis 30 %, bevorzugt von 2 % bis 20 % und besonders bevorzugt von 4 % bis 16 % eingesetzt.

In einer besonders bevorzugten Ausführungsform die mit den oben beschriebenen bevorzugten Ausführungsformen für die bevorzugten Konzentrationsbereiche identisch sein kann und bevorzugt identisch ist, enthalten die Flüssigkristallmedien

- eine oder mehrere Verbindungen der Formel Ia und
- eine oder mehrere Verbindungen der Formel II, bevorzugt ausgewählt aus der Gruppe der Verbindungen der Formeln II2 bis II4 und/oder
- eine oder mehrere Verbindungen der Formel III, bevorzugt ausgewählt ausgewählt aus der Gruppe der Verbindungen der Formeln III1 und III5.

Die erfindungsgemäßen Flüssigkristallmedien weisen bevorzugt nematische Phasen von jeweils mindestens von -10˚C bis 70 ˚C, bevorzugt von -30 ˚C bis 80 ˚C und ganz besonders bevorzugt von -40 ˚C bis 90 ˚C auf. Hierbei bedeutet der Begriff eine nematische Phase aufweisen einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, einer der elektrooptischen Anwendung entsprechenden Schichtdicke, für mindestens 100 Stunden überprüft. Bei hohen Temperaturen wird der Klärpunkt nach üblichen Methoden in Kapillaren gemessen. '

Außerdem weisen die erfindungsgemäßen Flüssigkristallmedien relativ kleine Werte für die Freedericksz-Schwellenspannung von kleiner oder gleich 3,0 V, bevorzugt kleiner oder gleich 2,0 V, besonders bevorzugt kleiner oder gleich 1,5 V und ganz besonders bevorzugt kleiner oder gleich 1,0 V auf.

Diese bevorzugten Werte für die einzelnen physikalischen Eigenschaften werden auch jeweils miteinander

kombiniert eingehalten. So weisen erfindungsgemäße Medien insbesondere die folgenden Eigenschaftskombinationen auf:

Der Ausdruck "Alkyl" umfaßt vorzugsweise geradkettige und verzweigte Alkylgruppen mit 1-7 Kohlenstoffatomen, insbesondere die geradkettigen Gruppen Methyl, Ethyl, Propyl, Butyl, Pentyl, Hexyl und Heptyl. Gruppen mit 2-5 Kohlenstoffatomen sind im allgemeinen bevorzugt.

Der Ausdruck "Alkenyl" umfaßt vorzugsweise geradkettige und verzweigte Alkenylgruppen mit 2-7 Kohlenstoffatomen, insbesondere die geradkettigen Gruppen. Besonders bevorzugte Alkenylgruppen sind $C_2$-$C_7$-1 E-Alkenyl, $C_4$-$C_7$-3E-Alkenyl, $C_5$-$C_7$-4-Alkenyl, $C_6$-$C_7$-5-Alkenyl und $C_7$-6-Alkenyl, insbesondere $C_2$-$C_7$-1E-Alkenyl, $C_4$-$C_7$-3E-Alkenyl und $C_5$-$C_7$-4-Alkenyl. Beispiele weiterer bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfaßt vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl", bzw. Alkoxyalkyl umfaßt vorzugsweise geradkettige Reste der Formel $C_nH_{2n+1}$-O-$(CH_2)_m$, worin n und m jeweils unabhängig voneinander 1 bis 6 bedeuten. Vorzugsweise ist n = 1 und m 1 bis 6.

In der vorliegenden Anmeldung bedeuten die Begriffe dielektrisch positive Verbindungen solche Verbindungen mit einem $\Delta\varepsilon > 1{,}5$, dielektrisch neutrale Verbindungen solche mit $-1{,}5 \leq \Delta\varepsilon \leq 1{,}5$ und dielektrisch negative Verbindungen solche mit $\Delta\varepsilon < -1{,}5$. Hierbei wird die dielektrische Anisotropie der Verbindungen bestimmt indem 10 % der Verbindungen in einem flüssigkristallinen Host gelöst werden und von dieser Mischung die Kapazität in mindestens jeweils einer Testzelle mit 10 $\mu$m Dichte mit homeotroper und mit homogener Oberflächenorientierung bei 1 kHz bestimmt wird. Die Meßspannung beträgt typischerweise 0,5 V bis 1,0 V, jedoch stets weniger als die kapazitive Schwelle der jeweiligen Flüssigkristallmischung.

Als Hostmischung wird für dielektrisch positive Verbindungen ZLI-4792 und für dielektrisch neutrale sowie dielektrisch negative Verbindungen ZLI-3086, beide von Merck KGaA, Deutschland, verwendet. Aus der Änderung der dielektrischen Suszeptibilitäten der Hostmischung nach Zugabe der zu untersuchenden Verbindung und Extrapolartion auf 100 % der eingesetzten Verbindung werden die Werte für die jeweiligen zu untersuchenden Verbindungen erhalten.

Der Begriff Schwellenspannung bezieht sich üblicherweise auf die optische Schwelle für 10 % relativen Kontrast ($V_{10}$).

In Bezug auf die Flüssigkristallmischungen mit negativer dielektrischer Anisotropie wird der Begriff Schwellenspannung in der vorliegenden Anmeldung jedoch für die kapazitive Schwellenspannung ($V_0$), auch Freedericksz-Schwelle genannt, verwendet, sofern nicht explizit anders angegeben.

Alle Konzentrationen in dieser Anmeldung, soweit nicht explizit anders vermerkt, sind in Massenprozent angegeben und beziehen sich auf die entsprechende Gesamtmischung. Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20 °C, sofern nicht explizit anders angegeben. $\Delta n$ wird bei 589 nm und $\Delta\varepsilon$ bei 1 kHz bestimmt. Die Schwellenspannungen sowie die anderen elektrooptischen Eigenschaften wurden in bei Merck KGaA, Deutschland, hergestellten Testzellen unter Verwendung von weißem Licht mit einem kommerziellen Meßgerät der Fa. Otsuka, Japan, bestimmt. Hierzu wurden Zellen je nach $\Delta n$ der Flüssigkristalle mit einer Dicke entsprechend einer optische Verzögerung $d\cdot\Delta n$ der Zellen von ca. 0,50 $\mu$m gewählt. Die Zellen wurden im sogenannten "normal hellen Modus" (Englisch "normally white mode") mit zu den Reiberichtungen an den benachbarten Substraten parallelen Polarisatoren betrieben. Die charakteristischen Spannungen wurden alle bei senkrechter Beobachtung bestimmt. Die Schwellenspannung wurde als $V_{10}$ für 10 % relativen Kontrast angegeben, die Mittgrau-spannung $V_{50}$ für 50 % relativen Kontrast und die Sättigungsspannung $V_{90}$ für 90 % relativen Kontrast.

Bei den Flüssigkristallmedien mit negativer dielektrischer Anisotropie wurde die Schwellenspannung als kapazitive Schwellung $V_0$ (auch Freedericksz-Schwelle genannt) in Zellen mit durch Lecithin homeotrop orientierter Flüssigkristallschicht bestimmt.

Die erfindungsgemäßen Flüssigkristallmedien können bei Bedarf auch weitere Zusatzstoffe und gegebenenfalls auch weitere chirale Dotierstoffe in den üblichen Mengen enthalten. Die eingesetzte Menge dieser Zusatzstoffe beträgt insgesamt 0 % bis 10 % bezogen auf die Menge der gesamten Mischung bevorzugt 0,1 % bis 6 %. Die Konzentration der einzelnen eingesetzten Verbindungen beträgt bevorzugt 0,1 bis 3 %. Die Konzentration dieser und ähnlicher Zusatzstoffe wird bei der Angabe der Konzentrationen sowie der Konzentrationsbereiche der Flüssigkristallverbindungen in den Flüssigkristallmedien nicht berücksichtigt.

Die Zusammensetzungen bestehen aus mehreren Verbindungen, bevorzugt aus 3 bis 30, besonders bevorzugt

aus 6 bis 20 und ganz besonders bevorzugt aus 10 bis 16 Verbindungen, die auf herkömmliche Weise gemischt werden. In der Regel wird die gewünschte Menge der in geringerer Menge verwendeten Komponenten in den Komponenten gelöst, den Hauptbestandteil ausmachenden, zweckmäßigerweise bei erhöhter Temperatur. Liegt die gewählte Temperatur über dem Klärpunkt des Hauptbestandteils, so ist die Vervollständigung des Lösungsvorgangs besonders leicht zu beobachten. Es ist jedoch auch möglich, die Flüssigkristallmischungen auf anderen üblichen Wegen, z.B. unter Verwendung von Vormischungen oder aus einem sogenannten "Multi Bottle Sytemen" herzustellen.

Mittels geeigneter Zusatzstoffe können die erfindungsgemäßen Flüssigkristallphasen derart modifiziert werden, daß sie in jeder bisher bekannt gewordenen Art von LCDs und insbesondere in ECB-Anzeigen, VA-Anzeigen, PA LCDs, ASM LCDs, sowie IPS-Anzeigen einsetzbar sind.

Die nachstehenden Beispiele dienen zur Veranschaulichung der Erfindung, ohne sie zu beschränken. In den Beispielen sind der Schmelzpunkt T (C,N), der Übergang von der smektischen (S) zur nematischen (N) Phase T(S,N) und Klärpunkt T (N,I) einer Flüssigkristallsubstanz in Grad Celsius angegeben. Die Prozentangaben sind, soweit nicht explizit anders gekennzeichnet, vor- und nachstehend Massenprozente und die physikalischen Eigenschaften sind die Werte bei 20 ˚C, sofern nicht explizit anders angegeben.

Alle angegebenen Werte für Temperaturen in dieser Anmeldung sind ˚C und alle Temperaturdifferenzen entsprechend Differenzgrad, sofern nicht explizit anders angegeben.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste $C_nH_{2n+1}$ und $C_mH_{2m+1}$ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten $R^1$, $R^2$, $L^1$ und $L^2$:

| Code für $R^1$, $R^2$, $L^1$, $L^2$ | $R^1$ | $R^2$ | $L^1$ | $L^2$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| n | $C_nH_{2n+1}$ | CN | H | H |
| nN.F | $C_nH_{2n+1}$ | CN | H | F |
| nN.F.F | $C_nH_{2n+1}$ | CN | F | F |
| nF | $C_nH_{2n+1}$ | F | H | H |
| nOF | $OC_nH_{2n+1}$ | F | H | H |
| nF.F | $C_nH_{2n+1}$ | F | H | F |
| nF.F.F | $C_nH_{2n+1}$ | F | F | F |
| nmF | $C_nH_{2n+1}$ | $C_mH_{2m+1}$ | F | H |
| $nCF_3$ | $C_nH_{2n+1}$ | $CF_3$ | H | H |
| $nOCF_3$ | $C_nH_{2n+1}$ | $OCF_3$ | H | H |
| $nOCF_3$.F | $C_nH_{2n+1}$ | $OCF_3$ | H | F |
| $nOCF_3$.F.F | $C_nH_{2n+1}$ | $OCF_3$ | F | F |
| $nOCF_2$.F | $C_nH_{2n+1}$ | $OCHF_2$ | H | H |
| $nOCF_2$.F | $C_nH_{2n+1}$ | $OCHF_2$ | H | F |
| $nOCF_2$.F.F | $C_nH_{2n+1}$ | $OCHF_2$ | F | F |
| nS | $C_nH_{2n+1}$ | NCS | H | H |
| rVsN | $C_rH_{2r+1}$-CH=CH-$C_sH_{2s}$- | CN | H | H |
| nEsN | $C_rH_{2r+1}$-O-$C_sH_{2s}$- | CN | H | H |
| nAm | $C_nH_{2n+1}$ | $COOC_mH_{2m+1}$ | H | H |
| nCl | $C_nH_{2n+1}$ | Cl | H | H |
| nCl.F | $C_nH_{2n+1}$ | Cl | H | F |
| nCl.F.F | $C_nH_{2n+1}$ | Cl | F | F |

**Tabelle A:**

| | |
|---|---|
| 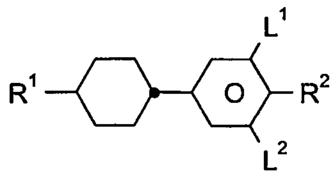<br>**PCH** | 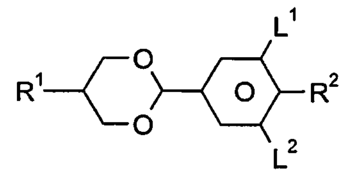<br>**PDX** |
| 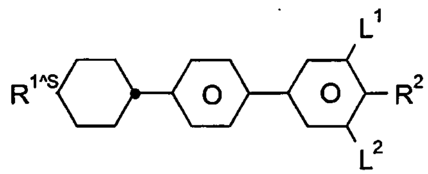<br>**BCH** | 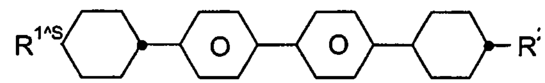<br>**CBC** |
| 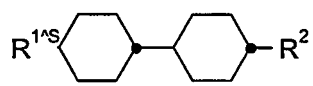<br>**CCH** | 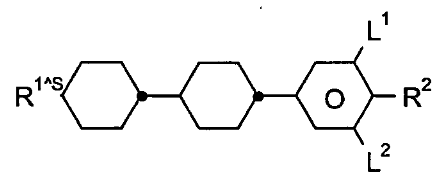<br>**CCP** |
| 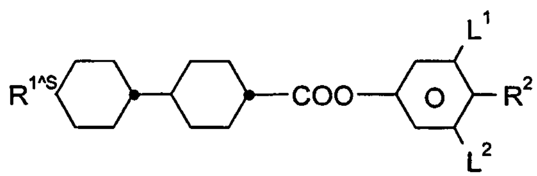<br>**CP** | 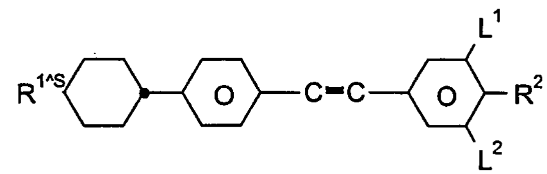<br>**CPTP** |

(fortgesetzt)

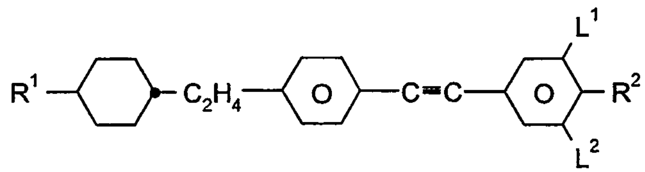

CEPTP

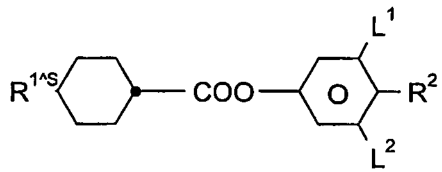

D

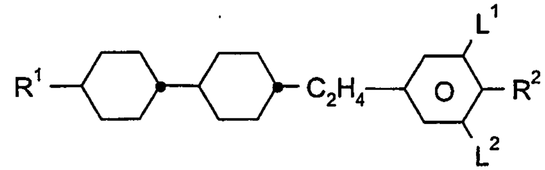

ECCP

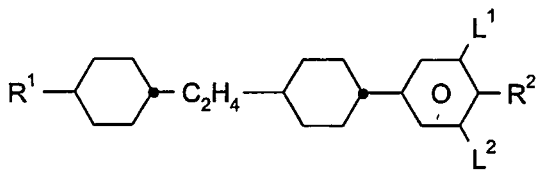

CECP

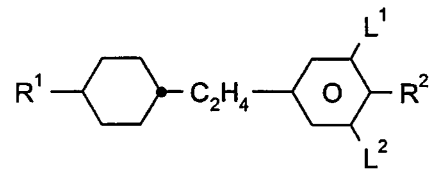

EPCH

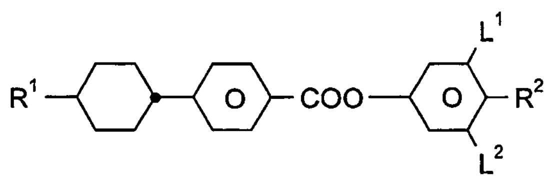

HP

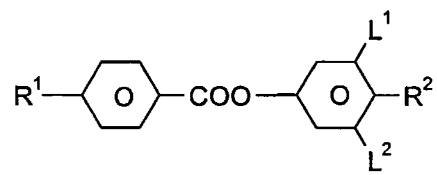

ME

(fortgesetzt)

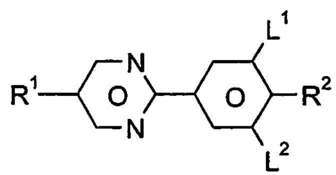

PYP

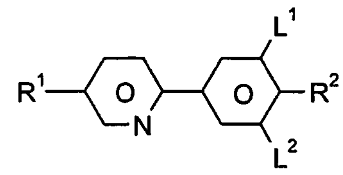

PYRP

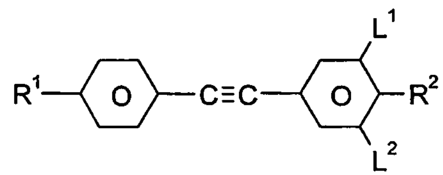

PTP

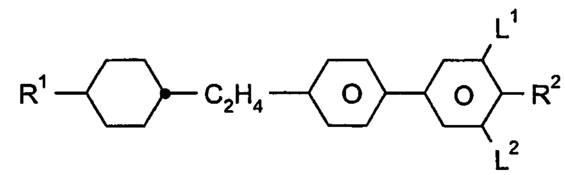

BECH

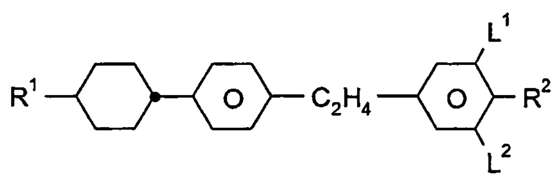

EBCH

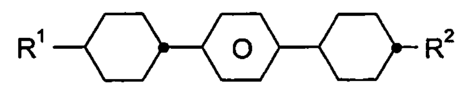

CPC

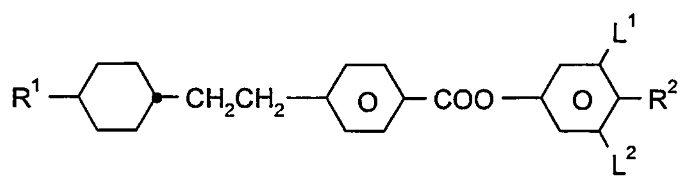

EHP

(fortgesetzt)

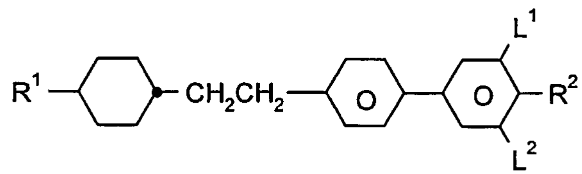

BEP

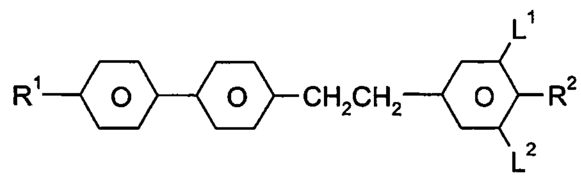

ET

**Tabelle B:**

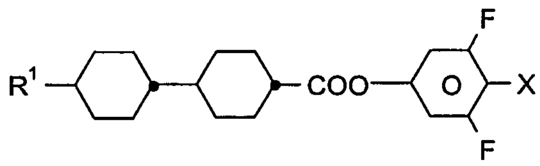

**CCZU-n-X**
(X=F, Cl, $-OCF_2H$, $-OCF_3$)

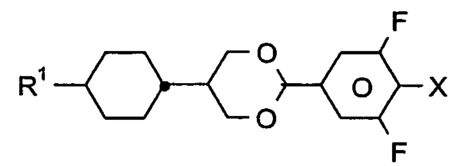

**CDU-n-X**
(X=F, Cl, $-OCF_2H$, $-OCF_3$)

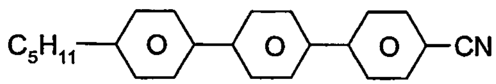

**T15**

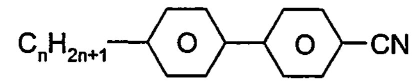

**K3n**

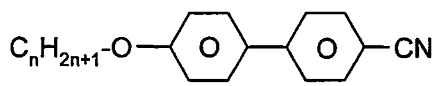

**M3n**

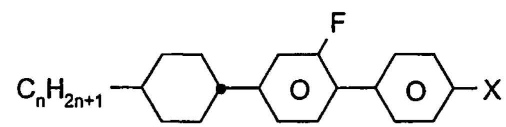

**CGP-n-X**
(X=F, Cl, $-OCF_2H$, $-OCF_3$)

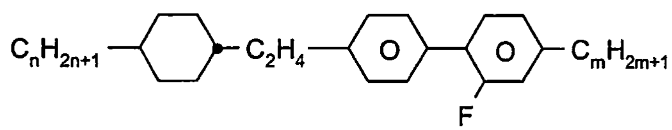

**Inm**

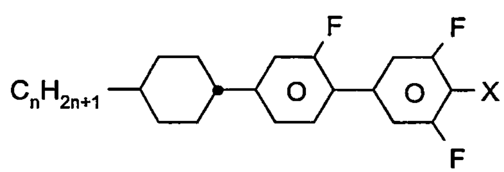

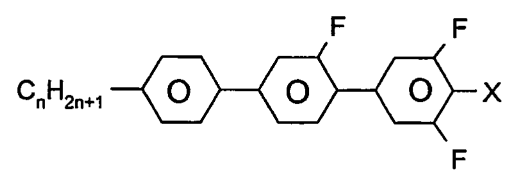

(fortgesetzt)

**CGU-n-X**
(X=F, Cl, $-OCF_2H$, $-OCF_3$)

**PGU-n-X**
(X=F, Cl, $-OCF_2H$, $-OCF_3$)

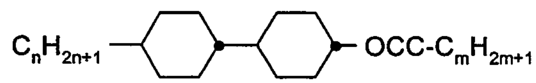

**C-nm**

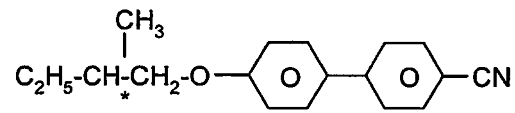

**C15**

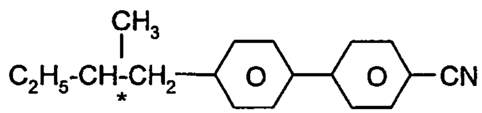

**CB15**

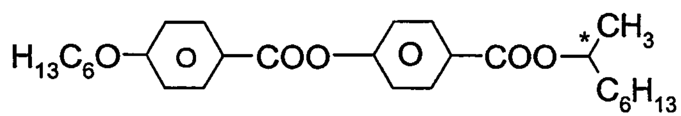

**S-811**

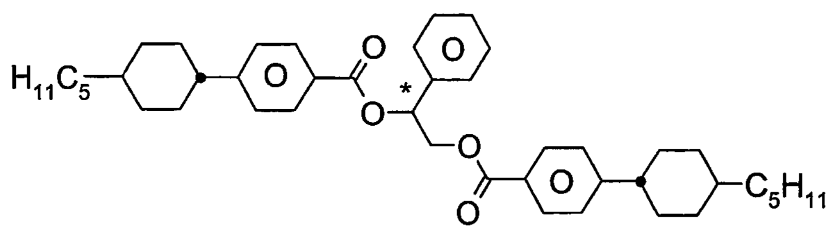

**S-1011**

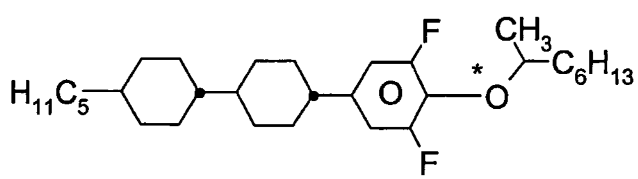

**S-2011**

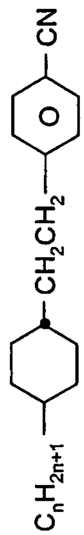

G3n

(fortgesetzt)

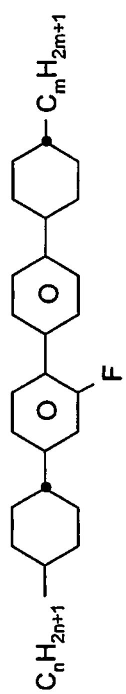

CBC-nmF

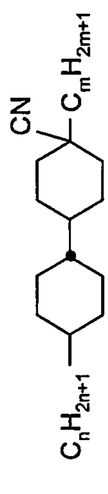

CCN-nm

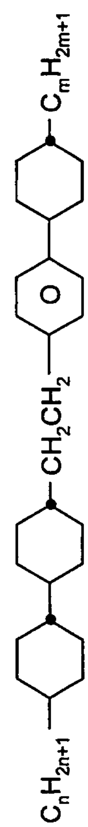

CCEPC-nm

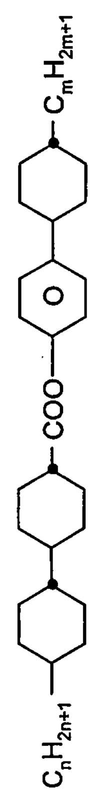

CCPC-nm

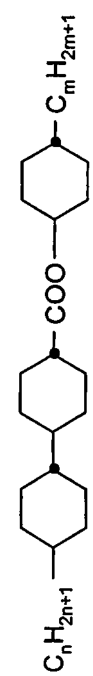

CH-nm

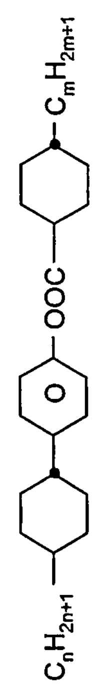

HD-nm

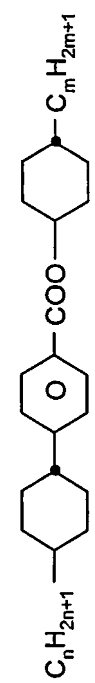

HH-nm

(fortgesetzt)

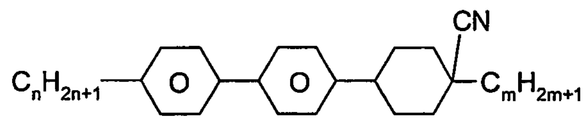

NCB-nm

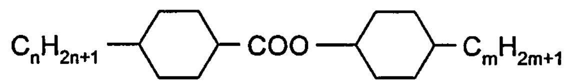

OS-nm

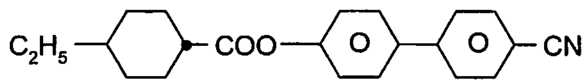

CHE

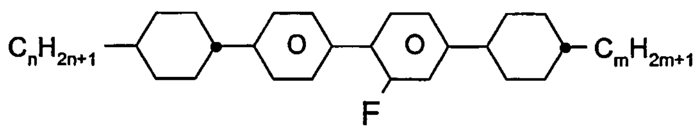

CBC-nmF

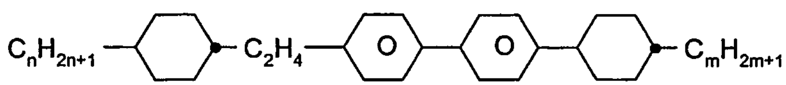

ECBC-nm

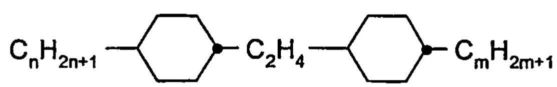

ECCH-nm

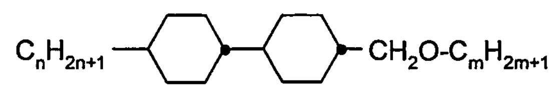

CCH-n1EM

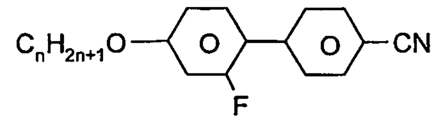

B-nO.FN

(fortgesetzt)

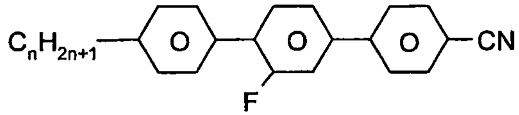

T-nFN

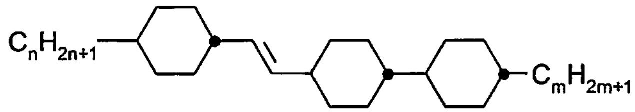

CVCC-n-m

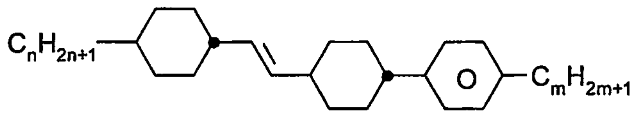

CVCP-n-m

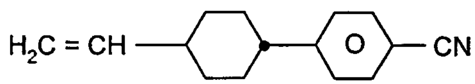

CP-V-N

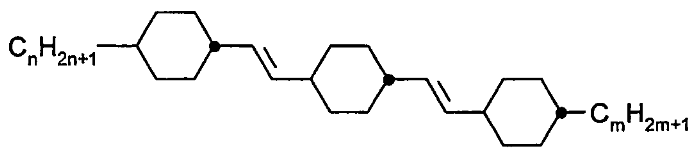

CVCVC-n-m

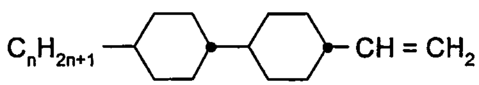

CC-n-V

(fortgesetzt)

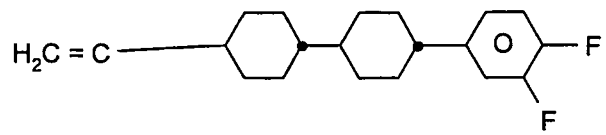

CCG-V-F

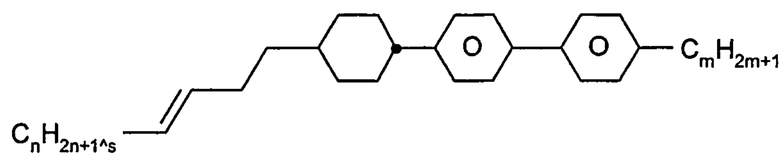

CPP-nV2-m

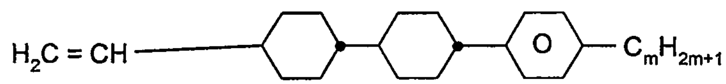

CCP-V-m

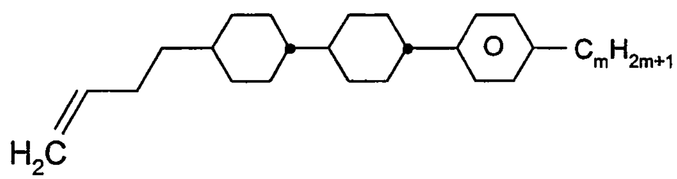

CCP-V2-m

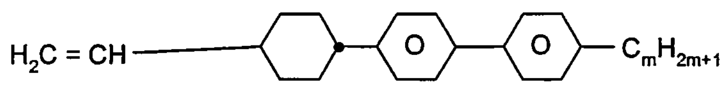

CPP-V-m

(fortgesetzt)

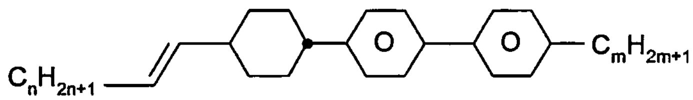

CPP-nV-m

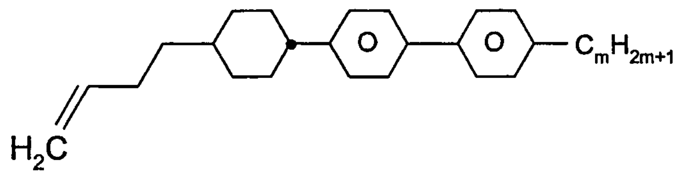

CPP-V2-m

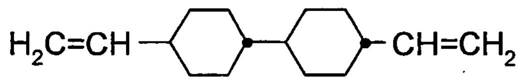

CC-V-V

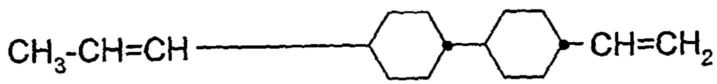

CC-1V-V

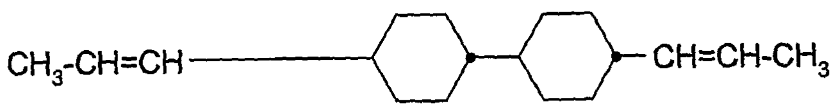

CC-1V-V1

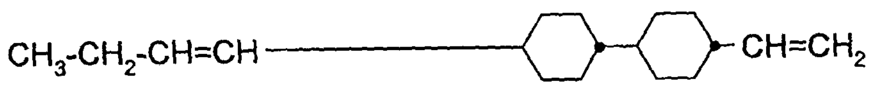

CC-2V-V

(fortgesetzt)

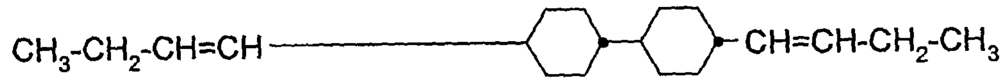

CC-2V-V2

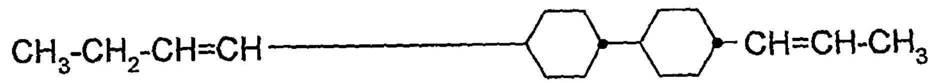

CC-2V-V1

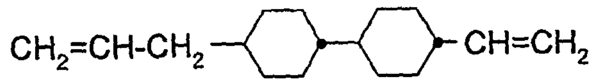

CC-V1-V

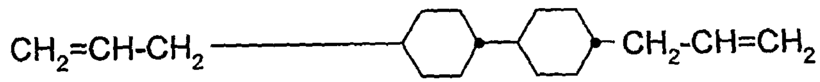

CC-V1-1V

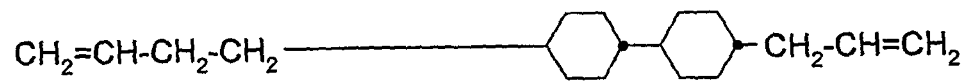

CC-V2-1V

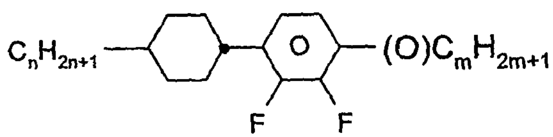

PCH-n(O)mFF

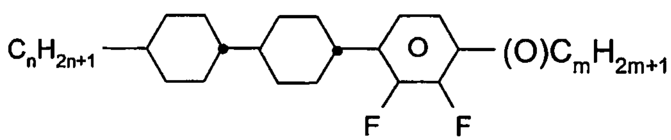

(fortgesetzt)

**CCP-n(O)mFF**

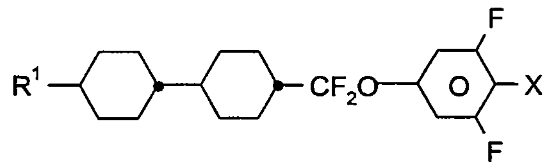

**CCQU-n-X**
(X=F, Cl, $-OCF_2H$, $-OCF_3$)

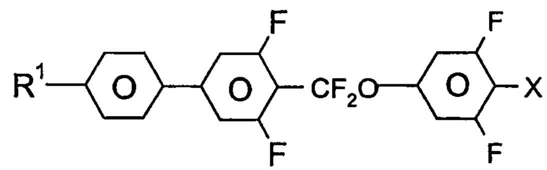

**PUQU-n-X**
(X=F, Cl, $-OCF_2H$, $-OCF_3$)

Beispiele

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. $\Delta n$ bedeutet optische Anisotropie (589 nm, 20 °C), $\Delta\varepsilon$ die dielektrische Anisotropie (1 kHz, 20 °C), H.R. die "Voltage Holding Ratio" (bei 100 °C, nach 5 Minuten im Ofen, 1 V), $V_{10}$, $V_{50}$ und $V_{90}$ die Schwellenspannung, Mittgrau-spannung bzw. Sättigungsspannung, sowie die kapazitive Schwellenspannung $V_0$ wurden bei 20 °C bestimmt.

Substanzbeispiele

Beispiel 1

Herstellung von chiralem 3,5-Di-tert.-butyl-3',5'-difluoro-4'-(1-methyl-heptyloxy)-biphenyl-4-ol

Stufe 1: Herstellung von 3,5-Difluoro-4-(1-methyl-heptyloxy)-brombenzol

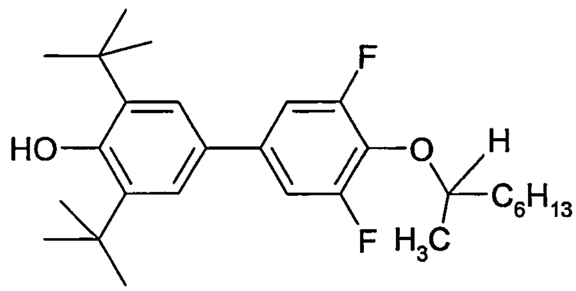

12,0 g 4-Brom-2,6-difluorphenol, 10,0 ml (S)-(+)-2-Octanol und 16,5 g Triphenylphosphin wurden bei ca. 20°C unter Rühren unter Stickstoffatmosphäre in 300 ml Tetrahydrofuran gelöst. Anschließend wurden 12,5 ml wasserfreies (max. 0,0075% $H_2O$) Diisopropylazodicarboxylat zugetropft. Die Reaktion verläuft exotherm. Die Geschwindigkeit der Zugabe wurde so gewählt, dass die Temperatur des Gemisches 45°C nicht überstieg. Anschließend wurde die Reaktionslösung 2 h gerührt und dann das Lösungsmittel im Rotationsverdampfer entfernt. Das Rohprodukt wurde in einer Mischung aus Chlorbutan und Heptan im Verhältnis 1:1 über 21 Kieselgel aufgereinigt. Es wurden 14,5 g 3,5-difluoro-4-(1-methyl-heptyloxy)-brombenzol als klare Flüssigkeit erhalten.

Stufe 2: Herstellung von 3,5-Difluoro-4-(1-methyl-heptyloxy)-phenylboronsäure

14,5 g 1-Brom-3,5-difluoro-4-(1-methyl-heptyloxy)-benzol aus Stufe 1 wurden in 150 ml Diethylether gelöst und auf -70°C gekühlt. Bei dieser Temperatur wurden unter Kühlung zunächst 31,0 ml einer 15%-igen Lösung von Butyllithium in n-Hexan langsam zugetropft, 1 h bei der selben Temperatur gerührt und dann mit 5,6 ml Trimethylborat langsam zugetropft und wieder h gerührt. Die Temperatur der Reaktionslösung wurde langsam auf -10°C ansteigen lassen. Dann wurde mit destilliertem Wasser hydrolysiert und der pH-Wert mit Salzsäure auf 2 eingestellt. Die organische Phase wurde abgetrennt und die wäßrige Phase mit MTB-Ether extrahiert. Das Produkt wurde wie üblich aufgereinigt und über $Na_2SO_4$ getrocknet. Es wurden 13,9 g 3,5-Difluoro-4-(1-methyl-heptyloxy)-phenylboronsäure erhalten.

Stufe 3: Herstellung von 3,5-Di-tert.-butyl-3',5'-difluoro-4'-(1-methyl-heptyloxy)-biphenyl-4-ol

3,5-Difluoro-4-(1-methyl-heptyloxy)-phenylboronsäure aus Stufe 2 wurden mit 12,0 g 2,6-Di-tert.-butyl-4-brom-phenol, 30 mg Palladium(II)-acetat und 100 mg Triphenylphosphin in 100 ml 2-Propanol gelöst und 30 ml 2-molarer wäßriger Natriumcarbonatlösung und 20 ml destilliertes Wasser zugesetzt. Dann wurde 15 h unter Rückfluß auf 80°C erhitzt. Dann wurde das Reaktionsgemisch auf ca. 20°C abgekühlt und mit Wasser verdünnt. Es wurde MBT-Ether zugegeben und die organische Phase abgetrennt. Das Produkt wurde wie üblich aufgereinigt.

Es wurden 8,4 g 3,5-Di-tert.-butyl-3',5'-difluoro-4'-(1-methyl-heptyloxy)-biphenyl-4-ol als farbloses Öl mit einer Glastemperatur von -21°C erhalten.

Beispiele 2 bis 54

Analog zu Beispiel 1 werden hergestellt:

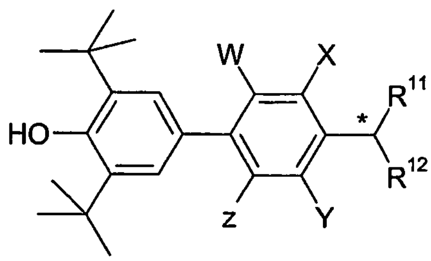

mit W und Z gleich H.

| Nr. | Konf.§ | $R^{11}$ | $R^{12}$ | X | Y | Eigenschaften |
|---|---|---|---|---|---|---|
| 2 | S | $C_2H_5$ | $CH_3$ | H | H | |
| 3 | S | $n$-$C_3H_7$ | $CH_3$ | H | H | |
| 4 | S | $n$-$C_4H_9$ | $CH_3$ | H | H | |
| 5 | S | $n$-$C_5H_{11}$ | $CH_3$ | H | H | |
| 6 | S | $n$- $C_6H_{13}$ | $CH_3$ | H | H | |
| 7 | S | $n$-$C_3H_7$ | $C_2H_5$ | H | H | |
| 8 | S | $n$-$C_4H_9$ | $C_2H_5$ | H | H | |
| 9 | S | $n$-$C_5H_{11}$ | $C_2H_5$ | H | H | |
| 10 | S | $n$- $C_6H_{13}$ | $C_2H_5$ | H | H | |
| 11 | S | $C_2H_5$ | $CH_3$ | F | H | |
| 12 | S | $n$-$C_3H_7$ | $CH_3$ | F | H | |
| 13 | S | $n$-$C_4H_9$ | $CH_3$ | F | H | |
| 14 | S | $n$-$C_5H_{11}$ | $CH_3$ | F | H | |
| 15 | S | $n$- $C_6H_{13}$ | $CH_3$ | F | H | |
| 16 | S | $n$-$C_3H_7$ | $C_2H_5$ | F | H | |
| 17 | S | $n$-$C_4H_9$ | $C_2H_5$ | F | H | |
| 18 | S | $n$-$C_5H_{11}$ | $C_2H_5$ | F | H | |
| 19 | S | $n$- $C_6H_{13}$ | $C_2H_5$ | F | H | |
| 20 | S | $C_2H_5$ | $CH_3$ | F | F | |
| 21 | S | $n$-$C_3H_7$ | $CH_3$ | F | F | |
| 22 | S | $n$-$C_4H_9$ | $CH_3$ | F | F | |
| 23 | S | $n$-$C_5H_{11}$ | $CH_3$ | F | F | |
| 1 | S | $n$- $C_6H_{13}$ | $CH_3$ | F | F | |
| 24 | S | $n$-$C_3H_7$ | $C_2H_5$ | F | F | |
| 25 | S | $n$-$C_4H_9$ | $C_2H_5$ | F | F | |
| 26 | S | $n$-$C_5H_{11}$ | $C_2H_5$ | F | F | |
| 27 | S | $n$- $C_6H_{13}$ | $C_2H_5$ | F | F | |
| 28 | R | $C_2H_5$ | $CH_3$ | H | H | |
| 29 | R | $n$-$C_3H_7$ | $CH_3$ | H | H | |
| 30 | R | $n$-$C_4H_9$ | $CH_3$ | H | H | |
| 31 | R | $n$-$C_5H_{11}$ | $CH_3$ | H | H | |
| 32 | R | $n$- $C_6H_{13}$ | $CH_3$ | H | H | |
| 33 | R | $n$-$C_3H_7$ | $C_2H_5$ | H | H | |
| 34 | R | $n$-$C_4H_9$ | $C_2H_5$ | H | H | |
| 35 | R | $n$-$C_5H_{11}$ | $C_2H_5$ | H | H | |
| 36 | R | $n$-$C_6H_{13}$ | $C_2H_5$ | H | H | |

(fortgesetzt)

| Nr. | Konf.§ | $R^{11}$ | $R^{12}$ | X | Y | Eigenschaften |
|---|---|---|---|---|---|---|
| 37 | R | $C_2H_5$ | $CH_3$ | F | H | |
| 38 | R | $n$-$C_3H_7$ | $CH_3$ | F | H | |
| 39 | R | $n$-$C_4H_9$ | $CH_3$ | F | H | |
| 40 | R | $n$-$C_5H_{11}$ | $CH_3$ | F | H | |
| 41 | R | $n$- $C_6H_{13}$ | $CH_3$ | F | H | |
| 42 | R | $n$-$C_3H_7$ | $C_2H_5$ | F | H | |
| 43 | R | $n$-$C_4H_9$ | $C_2H_5$ | F | H | |
| 44 | R | $n$-$C_5H_{11}$ | $C_2H_5$ | F | H | |
| 45 | R | $n$- $C_6H_{13}$ | $C_2H_5$ | F | H | |
| 46 | R | $C_2H_5$ | $CH_3$ | F | F | |
| 47 | R | $n$-$C_3H_7$ | $CH_3$ | F | F | |
| 48 | R | $n$-$C_4H_9$ | $CH_3$ | F | F | |
| 49 | R | $n$-$C_5H_{11}$ | $CH_3$ | F | F | |
| 50 | R | $n$- $C_6H_{13}$ | $CH_3$ | F | F | |
| 51 | R | $n$-$C_3H_7$ | $C_2H_5$ | F | F | |
| 52 | R | $n$-$C_4H_9$ | $C_2H_5$ | F | F | |
| 53 | R | $n$-$C_5H_{11}$ | $C_2H_5$ | F | F | |
| 54 | R | $n$- $C_6H_{13}$ | $C_2H_5$ | F | F | |

Bemerkung:§ Konfiguration des eingesetzten Alkohols.
Bemerkung:§ Konfiguration des eingesetzten Alkohols.

Beispiele 55 bis 72

Analog zu Beispiel 1 werden hergestellt:

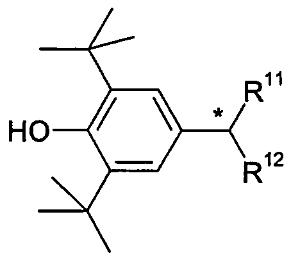

| Nr. | Konf.§ | $R^{11}$ | $R^{12}$ |
|---|---|---|---|
| 55 | S | $C_2H_5$ | $CH_3$ |
| 56 | S | $n$-$C_3H_7$ | $CH_3$ |
| 57 | S | $n$-$C_4H_9$ | $CH_3$ |
| 58 | S | $n$-$C_5H_{11}$ | $CH_3$ |
| 59 | S | $n$- $C_6H_{13}$ | $CH_3$ |
| 60 | S | $n$-$C_3H_7$ | $C_2H_5$ |
| 61 | S | $n$-$C_4H_9$ | $C_2H_5$ |
| 62 | S | $n$-$C_5H_{11}$ | $C_2H_5$ |
| 63 | S | $n$- $C_6H_{13}$ | $C_2H_5$ |
| 64 | R | $C_2H_5$ | $CH_3$ |
| 65 | R | $n$-$C_3H_7$ | $CH_3$ |
| 66 | R | $n$-$C_4H_9$ | $CH_3$ |
| 67 | R | $n$-$C_5H_{11}$ | $CH_3$ |

(fortgesetzt)

| Nr. | Konf.§ | $R^{11}$ | $R^{12}$ |
|---|---|---|---|
| 68 | R | $n$- $C_6H_{13}$ | $CH_3$ |
| 69 | R | $n$-$C_3H_7$ | $C_2H_5$ |
| 70 | R | $n$-$C_4H_9$ | $C_2H_5$ |
| 71 | R | $n$-$C_5H_{11}$ | $C_2H_5$ |
| 72 | R | $n$- $C_6H_{13}$ | $C_2H_5$ |

Bemerkung: § Konfiguration des eingesetzten Alkohols.

Beispiele 73 bis 126

Analog zu Beispiel 1 werden hergestellt:

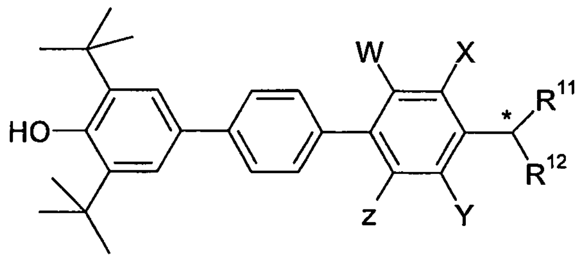

mit W und Z gleich H.

| Nr. | Konf.§ | $R^{11}$ | $R^{12}$ | X | Y |
|---|---|---|---|---|---|
| 73 | S | $C_2H_5$ | $CH_3$ | H | H |
| 74 | S | $n$-$C_3H_7$ | $CH_3$ | H | H |
| 75 | S | $n$-$C_4H_9$ | $CH_3$ | H | H |
| 76 | S | $n$-$C_5H_{11}$ | $CH_3$ | H | H |
| 77 | S | $n$- $C_6H_{13}$ | $CH_3$ | H | H |
| 78 | S | $n$-$C_3H_7$ | $C_2H_5$ | H | H |
| 79 | S | $n$-$C_4H_9$ | $C_2H_5$ | H | H |
| 80 | S | $n$-$C_5H_{11}$ | $C_2H_5$ | H | H |
| 81 | S | $n$- $C_6H_{13}$ | $C_2H_5$ | H | H |
| 82 | S | $C_2H_5$ | $CH_3$ | F | H |
| 83 | S | $n$-$C_3H_7$ | $CH_3$ | F | H |
| 84 | S | $n$-$C_4H_9$ | $CH_3$ | F | H |
| 85 | S | $n$-$C_5H_{11}$ | $CH_3$ | F | H |
| 86 | S | $n$- $C_6H_{13}$ | $CH_3$ | F | H |
| 87 | S | $n$-$C_3H_7$ | $C_2H_5$ | F | H |
| 88 | S | $n$-$C_4H_9$ | $C_2H_5$ | F | H |
| 89 | S | $n$-$C_5H_{11}$ | $C_2H_5$ | F | H |
| 90 | S | $n$- $C_6H_{13}$ | $C_2H_5$ | F | H |
| 91 | S | $C_2H_5$ | $CH_3$ | F | F |
| 92 | S | $n$-$C_3H_7$ | $CH_3$ | F | F |
| 92 | S | $n$-$C_4H_9$ | $CH_3$ | F | F |
| 94 | S | $n$-$C_5H_{11}$ | $CH_3$ | F | F |
| 95 | S | $n$- $C_6H_{13}$ | $CH_3$ | F | F |
| 96 | S | $n$-$C_3H_7$ | $C_2H_5$ | F | F |
| 97 | S | $n$-$C_4H_9$ | $C_2H_5$ | F | F |

(fortgesetzt)

| Nr. | Konf.§ | $R^{11}$ | $R^{12}$ | X | Y |
|---|---|---|---|---|---|
| 98 | S | $n$-$C_5H_{11}$ | $C_2H_5$ | F | F |
| 99 | S | $n$- $C_6H_{13}$ | $C_2H_5$ | F | F |
| 100 | R | $C_2H_5$ | $CH_3$ | H | H |
| 101 | R | $n$-$C_3H_7$ | $CH_3$ | H | H |
| 102 | R | $n$-$C_4H_9$ | $CH_3$ | H | H |
| 103 | R | $n$-$C_5H_{11}$ | $CH_3$ | H | H |
| 104 | R | $n$- $C_6H_{13}$ | $CH_3$ | H | H |
| 105 | R | $n$-$C_3H_7$ | $C_2H_5$ | H | H |
| 106 | R | $n$-$C_4H_9$ | $C_2H_5$ | H | H |
| 107 | R | $n$-$C_5H_{11}$ | $C_2H_5$ | H | H |
| 108 | R | $n$- $C_6H_{13}$ | $C_2H_5$ | H | H |
| 109 | R | $C_2H_5$ | $CH_3$ | F | H |
| 110 | R | $n$-$C_3H_7$ | $CH_3$ | F | H |
| 111 | R | $n$-$C_4H_9$ | $CH_3$ | F | H |
| 112 | R | $n$-$C_5H_{11}$ | $CH_3$ | F | H |
| 113 | R | $n$- $C_6H_{13}$ | $CH_3$ | F | H |
| 114 | R | $n$-$C_3H_7$ | $C_2H_5$ | F | H |
| 115 | R | $n$-$C_4H_9$ | $C_2H_5$ | F | H |
| 116 | R | $n$-$C_5H_{11}$ | $C_2H_5$ | F | H |
| 117 | R | $n$- $C_6H_{13}$ | $C_2H_5$ | F | H |
| 118 | R | $C_2H_5$ | $CH_3$ | F | F |
| 119 | R | $n$-$C_3H_7$ | $CH_3$ | F | F |
| 129 | R | $n$-$C_4H_9$ | $CH_3$ | F | F |
| 121 | R | $n$-$C_5H_{11}$ | $CH_3$ | F | F |
| 122 | R | $n$- $C_6H_{13}$ | $CH_3$ | F | F |
| 123 | R | $n$-$C_3H_7$ | $C_2H_5$ | F | F |
| 124 | R | $n$-$C_4H_9$ | $C_2H_5$ | F | F |
| 125 | R | $n$-$C_5H_{11}$ | $C_2H_5$ | F | F |
| 126 | R | $n$- $C_6H_{13}$ | $C_2H_5$ | F | F |

Bemerkung: § Konfiguration des eingesetzten Alkohols.

Beispiele 127 bis 180

Analog zu Beispiel 1 werden hergestellt:

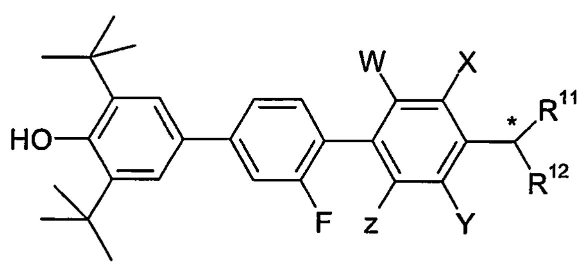

mit W und Z gleich H.

| Nr. | Konf.§ | $R^{11}$ | $R^{12}$ | X | Y |
|---|---|---|---|---|---|
| 127 | S | $C_2H_5$ | $CH_3$ | H | H |

(fortgesetzt)

| Nr. | Konf.§ | $R^{11}$ | $R^{12}$ | X | Y |
|---|---|---|---|---|---|
| 128 | S | *n*-$C_3H_7$ | $CH_3$ | H | H |
| 129 | S | *n*-$C_4H_9$ | $CH_3$ | H | H |
| 130 | S | *n*-$C_5H_{11}$ | $CH_3$ | H | H |
| 131 | S | *n*- $C_6H_{13}$ | $CH_3$ | H | H |
| 132 | S | *n*-$C_3H_7$ | $C_2H_5$ | H | H |
| 133 | S | *n*-$C_4H_9$ | $C_2H_5$ | H | H |
| 134 | S | *n*-$C_5H_{11}$ | $C_2H_5$ | H | H |
| 135 | S | *n*- $C_6H_{13}$ | $C_2H_5$ | H | H |
| 136 | S | $C_2H_5$ | $CH_3$ | F | H |
| 137 | S | *n*-$C_3H_7$ | $CH_3$ | F | H |
| 138 | S | *n*-$C_4H_9$ | $CH_3$ | F | H |
| 139 | S | *n*-$C_5H_{11}$ | $CH_3$ | F | H |
| 140 | S | *n*- $C_6H_{13}$ | $CH_3$ | F | H |
| 141 | S | *n*-$C_3H_7$ | $C_2H_5$ | F | H |
| 142 | S | *n*-$C_4H_9$ | $C_2H_5$ | F | H |
| 143 | S | *n*-$C_5H_{11}$ | $C_2H_5$ | F | H |
| 144 | S | *n*- $C_6H_{13}$ | $C_2H_5$ | F | H |
| 145 | S | $C_2H_5$ | $CH_3$ | F | F |
| 146 | S | *n*-$C_3H_7$ | $CH_3$ | F | F |
| 147 | S | *n*-$C_4H_9$ | $CH_3$ | F | F |
| 148 | S | *n*-$C_5H_{11}$ | $CH_3$ | F | F |
| 149 | S | *n*- $C_6H_{13}$ | $CH_3$ | F | F |
| 150 | S | *n*-$C_3H_7$ | $C_2H_5$ | F | F |
| 151 | S | *n*-$C_4H_9$ | $C_2H_5$ | F | F |
| 152 | S | *n*-$C_5H_{11}$ | $C_2H_5$ | F | F |
| 153 | S | *n*- $C_6H_{13}$ | $C_2H_5$ | F | F |
| 154 | R | $C_2H_5$ | $CH_3$ | H | H |
| 155 | R | *n*-$C_3H_7$ | $CH_3$ | H | H |
| 156 | R | *n*-$C_4H_9$ | $CH_3$ | H | H |
| 157 | R | *n*-$C_5H_{11}$ | $CH_3$ | H | H |
| 158 | R | *n*- $C_6H_{13}$ | $CH_3$ | H | H |
| 159 | R | *n*-$C_3H_7$ | $C_2H_5$ | H | H |
| 160 | R | *n*-$C_4H_9$ | $C_2H_5$ | H | H |
| 161 | R | *n*-$C_5H_{11}$ | $C_2H_5$ | H | H |
| 162 | R | *n*- $C_6H_{13}$ | $C_2H_5$ | H | H |
| 163 | R | $C_2H_5$ | $CH_3$ | F | H |
| 164 | R | *n*-$C_3H_7$ | $CH_3$ | F | H |
| 165 | R | *n*-$C_4H_9$ | $CH_3$ | F | H |
| 166 | R | *n*-$C_5H_{11}$ | $CH_3$ | F | H |
| 167 | R | *n*- $C_6H_{13}$ | $CH_3$ | F | H |
| 168 | R | *n*-$C_3H_7$ | $C_2H_5$ | F | H |
| 169 | R | *n*-$C_4H_9$ | $C_2H_5$ | F | H |
| 170 | R | *n*-$C_5H_{11}$ | $C_2H_5$ | F | H |
| 171 | R | *n*- $C_6H_{13}$ | $C_2H_5$ | F | H |
| 172 | R | $C_2H_5$ | $CH_3$ | F | F |
| 173 | R | *n*-$C_3H_7$ | $CH_3$ | F | F |
| 174 | R | *n*-$C_4H_9$ | $CH_3$ | F | F |
| 175 | R | *n*-$C_5H_{11}$ | $CH_3$ | F | F |
| 176 | R | *n*- $C_6H_{13}$ | $CH_3$ | F | F |
| 177 | R | *n*-$C_3H_7$ | $C_2H_5$ | F | F |

(fortgesetzt)

| Nr. | Konf.§ | $R^{11}$ | $R^{12}$ | X | Y |
|---|---|---|---|---|---|
| 178 | R | $n$-$C_4H_9$ | $C_2H_5$ | F | F |
| 179 | R | $n$-$C_5H_{11}$ | $C_2H_5$ | F | F |
| 180 | R | $n$- $C_6H_{13}$ | $C_2H_5$ | F | F |

Bemerkung: § Konfiguration des eingesetzten Alkohols.
Bemerkung: § Konfiguration des eingesetzten Alkohols.

Anwendunasbeispiele

Die Eigenschaften der erfindungsgemäßen Verbindungen werden untersucht. Dazu werden Flüssigkristallmischungen hergestellt und mit den entsprechenden Verbindungen versetzt.

Anwendunasbeispiel 1

Es wird eine nematische Flüssigkristallmischung (M0) der folgenden Zusammensetzung hergestellt, die die folgenden physikalischen Eigenschaften aufweist.

| Verbindung / Abkürzung | Konzentration / Massen-% | Physikalische Eigenschaften |
|---|---|---|
| PCH-5F | 8,0 | Klärpunkt (N,I) = 80,5 ˚C |
| PCH-6F | 6,4 | |
| PCH-7F | 4,8 | |
| $CCP\text{-}2OCF_3$ | 6,4 | |
| $CCP\text{-}3OCF_3$ | 9,6 | |
| $CCP\text{-}4OCF_3$ | 5,6 | |
| $CCP\text{-}5OCF_3$ | 8,8 | |
| BCH-3F.F | 9,6 | |
| BCH-5F.F | 8,0 | |
| $ECCP\text{-}3OCF_3$ | 4,0 | |
| $ECCP\text{-}3OCF_3$ | 4,0 | |
| CC-4-V | 20,0 | |
| CBC-33F | 1,6 | |
| CBC-53F | 1,6 | |
| CBC-55F | 1,6 | |
| Σ | 100,0 | |

Dieser Flüssigkristallmischung (M0) werden 0,5 % der Verbindung des Beispiels 1 zugesetzt. Dabei sinkt der Klärpunkt der Mischung (M1) auf 78,6˚C. Diese dotierte Mischung, M1 wird in einem offenen Kapillarrohr (ME-18 552 der Firma Mettler) bei einer Füllhöhe von 1 cm, in Gegenwart von Luftsauerstoff auf 150˚C erhitzt. Nach vorgegebenen Zeiten wird der Klärpunkt der Probe im Kapillarrohr bestimmt. Danach wird das Kapillarruhr wieder auf 150˚C erhitzt. Zum Vergleich wird die selbe Untersuchung an der nicht dotierten Mischung, M0 ausgeführt. Die Ergebnisse dieser Untersuchung sind in der folgenden Tabelle zusammengestellt.

| Mischung | M0 | M1 |
|---|---|---|
| c(B1) / % | 0 | 0,5 |
| \|P\| / μ m | ∞* | 74 |
| HTP / $\mu m^{-1}$ | n.a. | 2,7 |
| t / h | T(N,I) / ˚C | |
| 0 | 80,5 | 78,6 |
| 1 | 79,4 | 78,9 |

(fortgesetzt)

| Mischung | M0 | M1 |
|---|---|---|
| 2 | 79,5 | 78,8 |
| 5 | 76,8 | 78,8 |
| 10 | 74,0 | 78,9 |
| 30 | 63,7 | 78,7 |
| 50 | 59,9 | 78,4 |
| Bemerkungen: * nicht bestimmbar<br>n.a. nicht anwendbar. | | |

Die Meßgenauigkeit bei der Bestimmung des Klärpunkts betrug +/-0,3 Grad.

Die Mischung M1 hat hervorragende anwendungstechnische Eigenschaften und zeichnet sich insbesondere durch eine sehr gute thermische Stabilität, insbesondere bei Gegenwart von Luftsauerstoff aus.

Bei der undotierten Mischung (M0) sinkt der Klärpunkt in dem oben beschriebenen Stabilitätstest innerhalb von etwas mehr als vier Tagen um mehr als zwanzig Differenzgrad. Im Gegensatz dazu sinkt der Klärpunkt der erfindungsgemäßen Mischung nur unwesentlich, um 0,2 Differenzgrad.

Vergleichsbeispiel V 1

Der unter Anwendungsbeispiel 1 beschrieben Mischung, M0 werden 0,5 % des chiralen Dotierstoffs S-811 der Firma Merck KGaA zugesetzt. Die resultierende Mischung, V1 hat einen Klärpunkt von 79,0˚C und einen cholesterischen Pitch von 17 µm. Bei der unter Anwendungsbeispiel 1 beschriebenen, thermischen Belastung sinkt der Klärpunkt der Mischung

V1 deutlich innerhalb von 50 h auf 60,0˚C ab.

Vergleichsbeispiel V 2

Der unter Anwendungsbeispiel 1 beschriebenen Mischung, M0 werden 0,5 % des achiralen Phenols AP

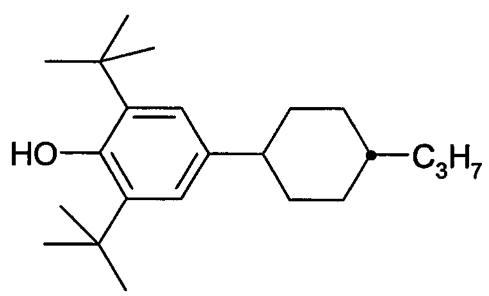

AP

der DE 195 391 41 mit einem Schmelzpunkt von 91 ˚C zugesetzt. Die resultierende Mischung, V2 hat einen Klärpunkt von 78,2˚C. Bei der unter Anwendungsbeispiel 1 beschriebenen, thermischen Belastung sinkt der Klärpunkt der Mischung V2 nur unwesentlich, auf 78,0˚C, ab. Da die zugesetzte Verbindung achiral ist, ist auch die Mischung V2 nicht chiral und hat somit keinen cholesterischen Pitch.

Diese Ergebnisse sind in der folgenden Tabelle zusammengestellt.

| Beispiel | -/- | Vergleichsbeispiele 1 und 2 | | Anwendungsbeispiel 1 |
|---|---|---|---|---|
| Mischung | M0 | V1 | V2 | M1 |
| Dotierstoff | -/- | S-811 | AP | B1 |
| \|P\| / µm | ∞* | 17 | ∞* | 74 |

(fortgesetzt)

| Beispiel | -/- | Vergleichsbeispiele 1 und 2 | | Anwendungsbeispiel 1 |
|---|---|---|---|---|
| $\Delta T^{\S}$ / ° | 20,6 | 19,0 | 0,2 | 0,2 |
| Bemerkungen: * nicht bestimmbar, § $\Delta T \equiv T(N,I),(50\ h) - T(N,I),(0\ h)$ | | | | |

**Patentansprüche**

**1.** Chirale Verbindung der Formel I

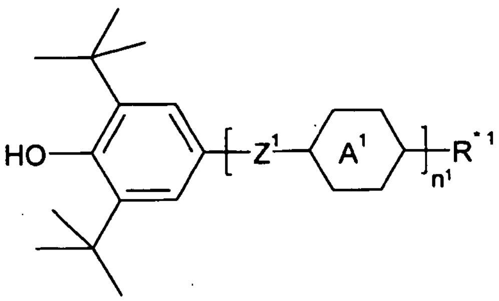

I

worin

$R^{*1}$ einen chiralen Rest der Formel I*,

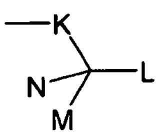

I*

bedeutet, worin

K eine Einfachbindung, Alkylen mit 1 bis 9, bevorzugt mit 1 bis 5 C-Atomen, Alkenylen oder Alkinylen mit 2 bis 9, bevorzugt mit 2 bis 5 C-Atomen, wobei bei allen drei Arten von Gruppen jeweils eine, zwei oder mehr der vorhandenen $-CH_2-$ Gruppen durch -O-, -C=O- oder -S- ersetzt sein können, wobei jedoch keine zwei O-Atome direkt miteinander verbunden sind und alle drei Arten von Gruppen optional durch Halogen substituiert sein können, und

L, M und N, jeweils unabhängig voneinander, aber verschieden voneinander und vom Rest des Moleküls inklusive der Gruppe K, Wasserstoff, Halogen, bevorzugt F, Aryl oder Cycloalkyl, Alkyl oder Alkoxy mit 1 bis 11 C-Atomen, Alkenyl, Alkenyloxy, Alkinyl oder Alkinyloxy mit 2 bis 11 C-Atomen, wobei bei allen sechs Arten von Gruppen jeweils eine, zwei oder mehr der vorhandenen $-CH_2-$ Gruppen durch -O-, -C=O- oder -S- ersetzt sein können, wobei jedoch keine zwei O-Atome direkt miteinander verbunden sind und alle sechs Arten von Gruppen optional durch Halogen substituiert sein können,

$Z^1$, wenn mehrfach vorhanden jeweils unabhängig voneinander, $-CH_2\text{-}CH_2-$, -CH=CH-, -C≡C-, -COO-, -OCO-, $-CH_2O-$, $-OCH_2-$, $-CF_2O-$, $-OCF_2-$, $-(CH_2)_4-$, -CF=CF-, -CH=CF-, -CF=CH-, $-CH_2-$, $-CF_2-$, -CHF-, -O-, -S- oder eine Einfachbindung,

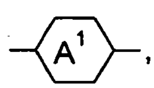,

wenn mehrfach vorhanden jeweils unabhängig voneinander,

(a) einen trans-1,4-Cyclohexylenrest, worin auch eine oder mehrere nicht benachbarte $CH_2$-Gruppen durch -O- und/oder -S- ersetzt sein können,
(b) einen 1,4-Cyclohexenylenrest,
(c) einen 1,4-Phenylenrest, worin auch eine oder zwei CH-Gruppen durch N ersetzt sein können oder
(d) einen Rest ausgewählt aus der Gruppe
1,4-Bicyclo-(2,2,2)-octylen,
Piperidin-1,4-diyl, Naphtalin-2,6-diyl,
Decahydronaphthalin-2,6-diyl und
1,2,3,4-Tetrahydronaphthalin-2,6-diyl,

wobei diese Reste (a) bis (d) und der phenolische Benzolring optional einfach oder mehrfach durch F-Atome substituiert sein können, und

$n^1$ 0, 1, 2 oder 3

bedeuten,
**dadurch gekennzeichnet, dass** sie in einem nematischen Flüssigkristall eine cholesterische Phase induziert und gleichzeitig als Stabilisator wirkt.

**2.** Verbindung nach Anspruch 1, der Formel Ia

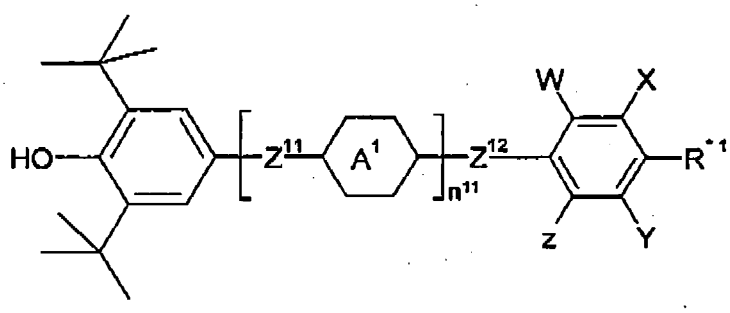

Ia

worin

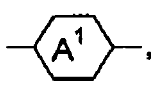

und $R^{*1}$ die in Anspruch 1 gegebene Bedeutung und
$Z^{11}$ und $Z^{12}$; jeweils unabhängig voneinander, die in Anspruch 1 für $Z^1$ gegebene Bedeutung

haben,und

$n^{11}$ 0, 1 oder 2 und
W, X, Y und Z, jeweils unabhängig voneinander, H, F, Cl, Alkyl, oder Alkoxy, bevorzugt mit 1 bis 7 C-Atomen

bedeuten.

**3.** Verbindung nach einem der Ansprüche 1 und 2, **dadurch gekennzeichnet, dass**

$R^{*1}$ einen chiralen Rest der Formel I*

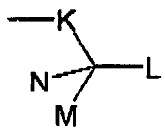

I*

bedeutet, worin

K eine Einfachbindung, Alkylen mit 1 bis 5 C-Atomen, Alkenylen oder Alkinylen mit 2 bis 5 C-Atomen, wobei bei allen drei Arten von Gruppen jeweils eine, zwei oder mehr der vorhandenen $-CH_2-$ Gruppen durch -O-, -C=O- oder -S- ersetzt sein können, wobei jedoch keine zwei O-Atome direkt miteinander verbunden sind und alle drei Arten von Gruppen optional durch Fluor substituiert sein können, und

L, M und N, jeweils unabhängig voneinander, aber verschieden voneinander und vom Rest des Moleküls inklusive der Gruppe K, Wasserstoff, F, Aryl oder Cycloalkyl, Alkyl oder Alkoxy 1 bis 7 C-Atomen, Alkenyl, Alkenyloxy, Alkinyl oder Alkinyloxy mit 2 bis 7 C-Atomen, wobei bei allen sechs Arten von Gruppen jeweils eine, zwei oder mehr der vorhandenen $-CH_2-$Gruppen durch -O-, -C=O- oder -S- ersetzt sein können, wobei jedoch keine zwei O-Atome direkt miteinander verbunden sind und alle sechs Arten von Gruppen optional durch Fluor substituiert sein können,

bedeuten.

**4.** Verbindung nach mindestens einem der Ansprüche 1 bis 3, **dadurch gekennzeichnet, dass**

$R^{*1}$ einen chiralen Rest ausgewählt aus der Gruppe der Reste der Formeln

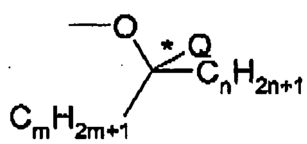

,

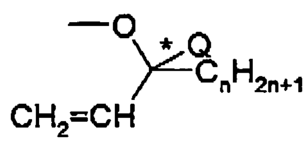

,

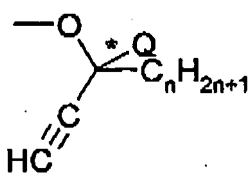

,

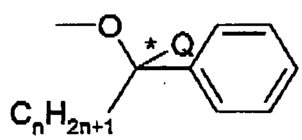

,

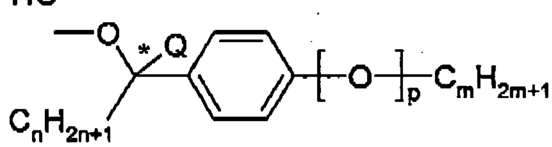

,

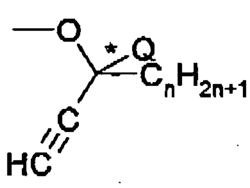

,

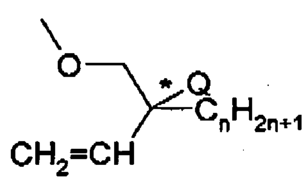

,

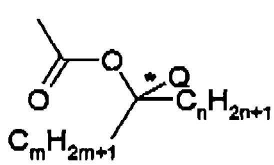

und

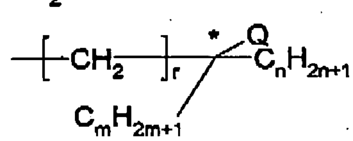

bedeutet, worin

Q H oder Halogen, bevorzugt H oder F, bevorzugt H,
n und m voneinander verschieden sind und ansonsten voneinander unabhängig 1 bis 11,
p 0 oder 1 und
r 0 bis 4

bedeuten.

**5.** Verbindung nach Anspruch 4, **dadurch gekennzeichnet, dass**

r 0 bis 2

bedeutet.

**6.** Verbindung nach mindestens einem der Ansprüche 1 bis 5, ausgewählt aus der Gruppe der Verbindungen der Formeln Ia-1 bis Ia-9

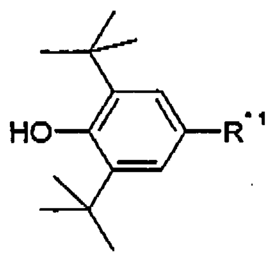

Ia-1

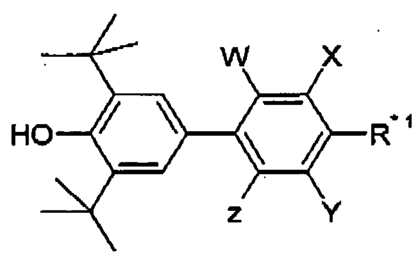

Ia-2

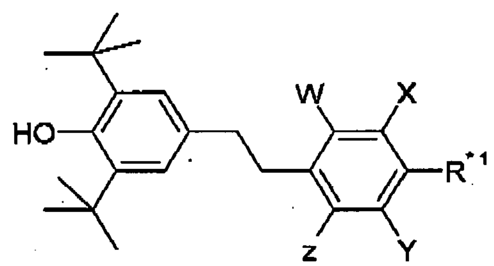

Ia-3

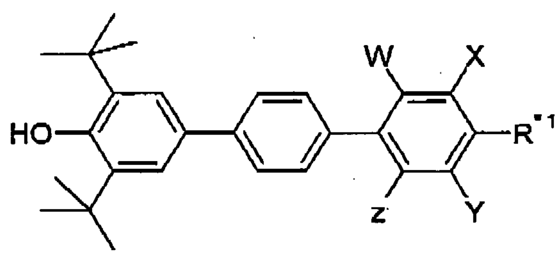

Ia-4

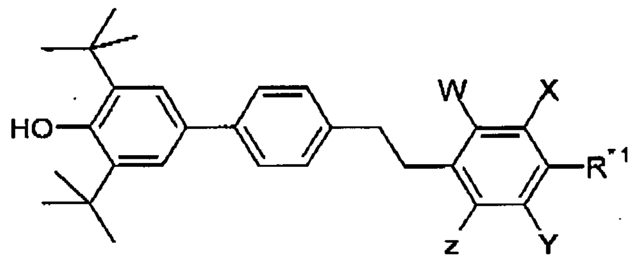

Ia-5

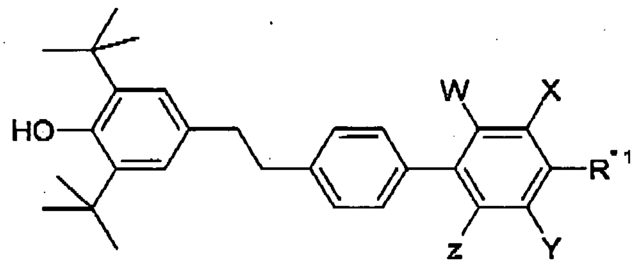

Ia-6

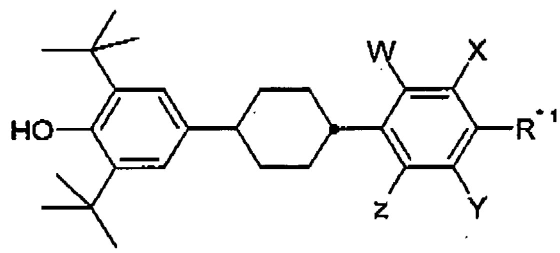

Ia-7

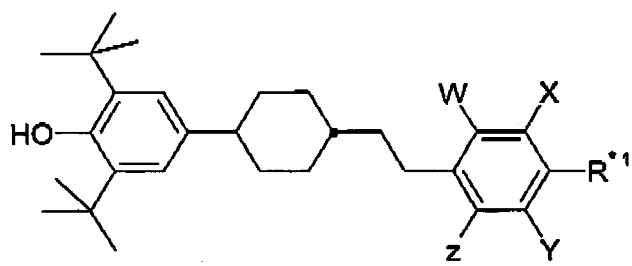

Ia-8

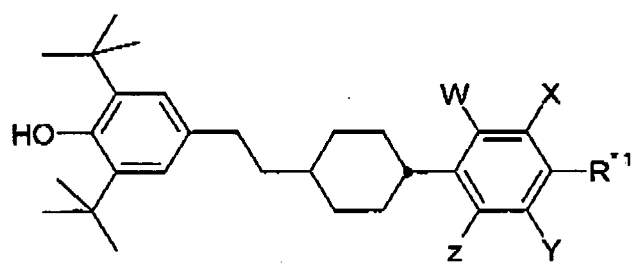

Ia-9

worin die Parameter die in Anspruch 2 gegebene Bedeutung haben.

7. Verbindung nach Anspruch 6, **dadurch gekennzeichnet, dass** W und Z H bedeuten.

**8.** Verwendung einer oder mehrerer Verbindungen nach mindestens einem der Ansprüche 1 bis 7 in einer Flüssigkristallmischung als chiraler Dotierstoff, als Stabilisator oder als chiraler Dotierstoff und gleichzeitig als Stabilisator.

**9.** Flüssigkristallmedium, **dadurch gekennzeichnet, dass** es eine oder mehrere Verbindungen nach mindestens einem der Ansprüche 1 bis 7 enthält.

**10.** Verwendung eines Flüssigkristallmediums nach Anspruch 9 in einer elektrooptischen Anzeige.

**11.** Elektrooptischen Anzeige enthaltend ein Flüssigkristallmedium nach Anspruch 9.

**12.** Verfahren zur Herstellung einer Flüssigkristallmischurtg, **dadurch gekennzeichnet, dass** der Flüssigkristallmischung eine Verbindung zugegeben wird, die eine cholesterische Phase induzieren und gleichzeitig als Stabilisator wirken.

**13.** Flüssigkristallmischung, **dadurch gekennzeichnet, dass** sie nach dem Verfahren nach Anspruch 12 hergestellt wurde.

## Claims

**1.** Chiral compound of the formula I

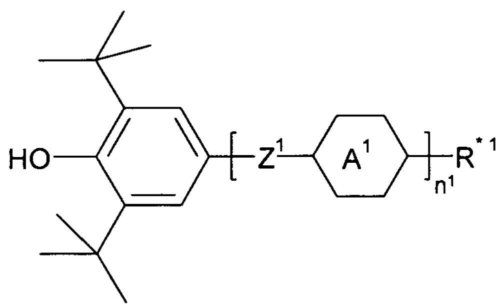

I

in which

$R^{*1}$ denotes a chiral radical of the formula I*

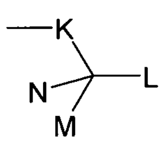

I*

in which

K denotes a single bond, alkylene having 1 to 9, preferably having 1 to 5 C atoms, alkenylene or alkynylene having 2 to 9, preferably having 2 to 5 C atoms, where one, two or more of the $-CH_2-$ groups present in all three types of group may each be replaced by -O-, -C=O- or -S-, but where no two O atoms are bonded directly to one another and all three types of group may optionally be substituted by halogen, and

L, M and N each, independently of one another, but differently from one another and from the remainder of the molecule including the group K, denote hydrogen, halogen, preferably F, aryl or cycloalkyl, alkyl or alkoxy having 1 to 11 C atoms, alkenyl, alkenyloxy, alkynyl or alkynyloxy having 2 to 11 C atoms, where one, two or more of the $-CH_2-$ groups present in all six types of group may each be replaced by -O-, -C=O- or -S-, but where no two O atoms are bonded directly to one another and all six types of group may optionally be substituted by halogen,

$Z^1$, if present more than once, in each case, independently of one another, denotes $-CH_2-CH_2-$, -CH=CH-,

-C≡C-, -COO-, -OCO-, $-CH_2O-$, $-OCH_2-$, $-CF_2O-$, $-OCF_2-$, $-(CH_2)_4-$, -CF=CF-, -CH=CF-, -CF=CH-, $-CH_2-$, $-CF_2-$, -CHF-, -O-, -S- or a single bond,

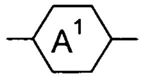,

if present more than once, in each case, independently of one another, denotes
(a) a trans-1,4-cyclohexylene radical, in which, in addition, one or more non-adjacent $CH_2$ groups may be replaced by -O- and/or -S-,
(b) a 1,4-cyclohexenylene radical,
(c) a 1,4-phenylene radical, in which, in addition, one or two CH groups may be replaced by N, or
(d) a radical selected from the group
1,4-bicyclo[2.2.2]octylene,
piperidine-1,4-diyl, naphthalene-2,6-diyl,
decahydronaphthalene-2,6-diyl and
1,2,3,4-tetrahydronaphthalene-2,6-diyl,

where these radicals (a) to (d) and the phenolic benzene ring may optionally be mono- or polysubstituted by F atoms, and

$n^1$ denotes 0, 1, 2 or 3,

**characterised in that** it induces a cholesteric phase in a nematic liquid crystal and simultaneously acts as stabiliser.

**2.** Compound according to Claim 1, of the formula Ia

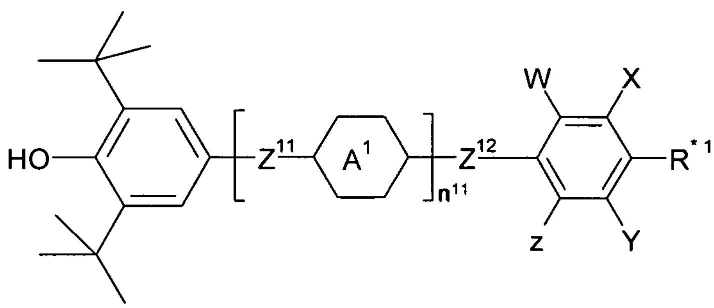

Ia

in which

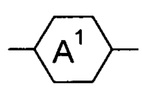

and $R^{*1}$ have the meaning given in Claim 1, and
$Z^{11}$ and $Z^{12}$ each, independently of one another, have the meaning given for $Z^1$ in Claim 1,

and

$n^{11}$ denotes 0, 1 or 2, and
W, X, Y and Z each, independently of one another, denote H, F, Cl, alkyl or alkoxy, preferably having 1 to 7 C atoms.

**3.** Compound according to one of Claims 1 and 2, **characterised in that**

$R^{*1}$ denotes a chiral radical of the formula I*

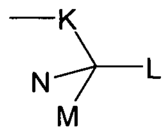

I*

in which
K denotes a single bond, alkylene having 1 to 5 C atoms, alkenylene or alkynylene having 2 to 5 C atoms, where one, two or more of the $-CH_2-$ groups present in all three types of group may each be replaced by -O-, -C=O- or -S-, but where no two O atoms are bonded directly to one another and all three types of group may optionally be substituted by fluorine, and
L, M and N each, independently of one another, but differently from one another and from the remainder of the molecule including the group K, denote hydrogen, F, aryl or cycloalkyl, alkyl or alkoxy having 1 to 7 C atoms, alkenyl, alkenyloxy, alkynyl or alkynyloxy having 2 to 7 C atoms, where one, two or more of the $-CH_2-$ groups present in all six types of group may each be replaced by -O-, -C=O- or -S-, but where no two O atoms are bonded directly to one another and all six types of group may optionally be substituted by fluorine.

**4.** Compound according to at least one of Claims 1 to 3, **characterised in that**

$R^{*1}$ denotes a chiral radical selected from the group of the radicals of the formulae

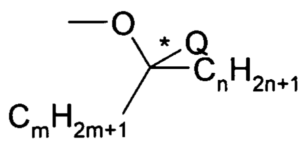

,

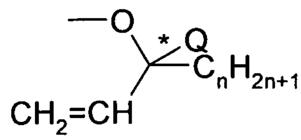

,

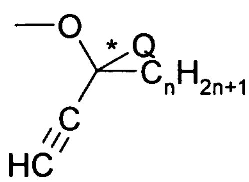

,

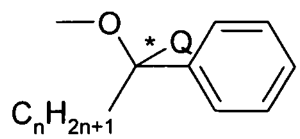

,

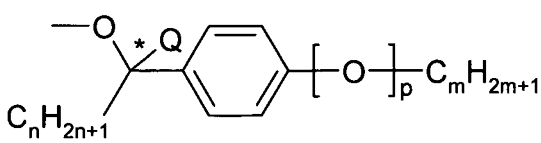

,

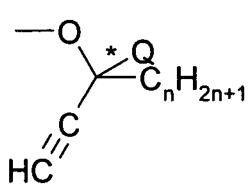

,

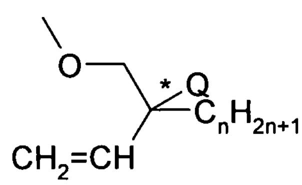

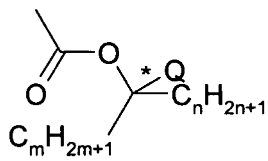

and

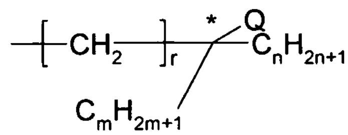

in which

Q denotes H or halogen, preferably H or F, preferably H,
n and m are different from one another and otherwise, independently of one another, denote 1 to 11,
p denotes 0 or 1, and
r denotes 0 to 4.

**5.** Compound according to Claim 4, **characterised in that**

r denotes 0 to 2.

**6.** Compound according to at least one of Claims 1 to 5, selected from the group of the compounds of the formulae Ia-1 to Ia-9

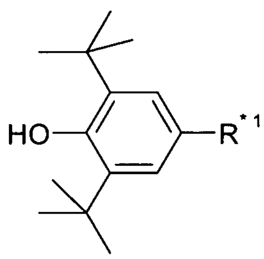

Ia-1

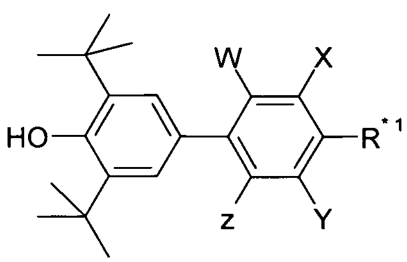

Ia-2

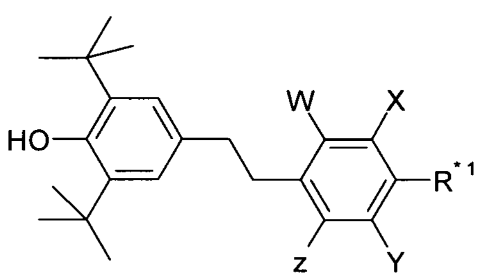

Ia-3

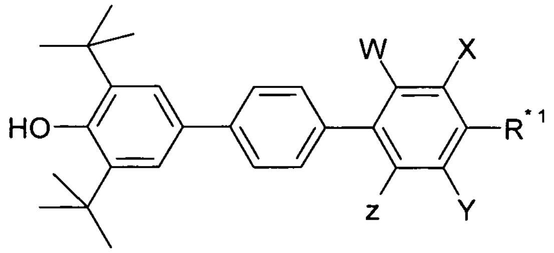

Ia-4

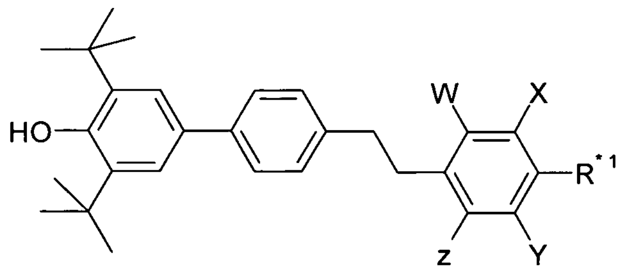

Ia-5

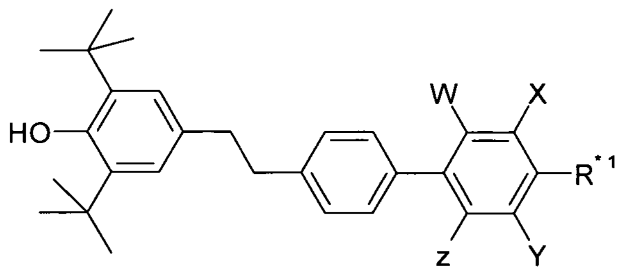

Ia-6

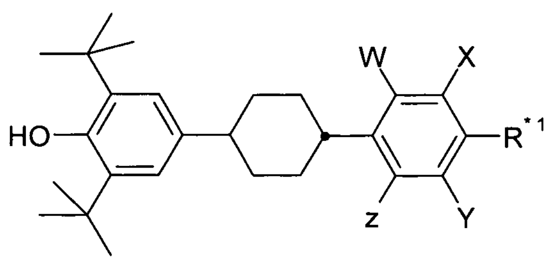

Ia-7

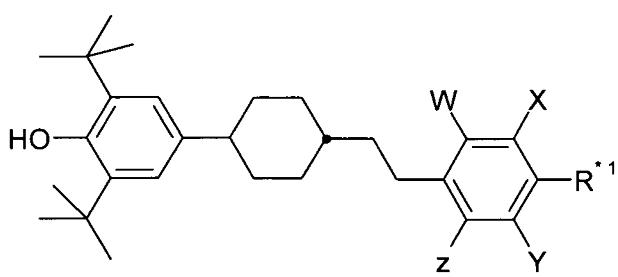

Ia-8

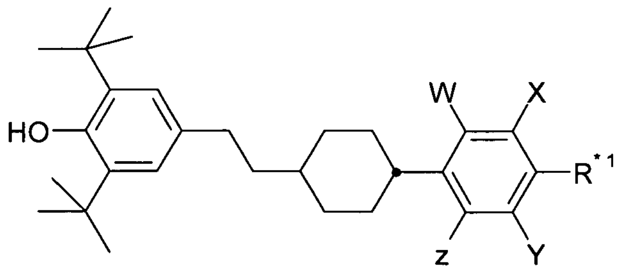

Ia-9

in which the parameters have the meaning given in Claim 2.

7. Compound according to Claim 6, **characterised in that** W and Z denote H.

8. Use of one or more compounds according to at least one of Claims 1 to 7 in a liquid-crystal mixture as chiral dopant, as stabiliser or as chiral dopant and simultaneously as stabiliser.

9. Liquid-crystal medium, **characterised in that** it comprises one or more compounds according to at least one of Claims 1 to 7.

10. Use of a liquid-crystal medium according to Claim 9 in an electro-optical display.

11. Electro-optical display containing a liquid-crystal medium according to Claim 9.

12. Process for the preparation of a liquid-crystal mixture, **characterised in that** a compound which induces a cholesteric phase and simultaneously acts as stabiliser is added to the liquid-crystal mixture.

13. Liquid-crystal mixture, **characterised in that** it has been prepared by the process according to Claim 12.

**Revendications**

1. Chiral composé de la formule I

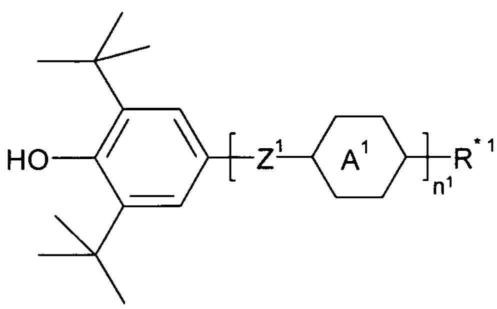

I

dans laquelle

$R^{*1}$ représente un radical chiral de la formule I*

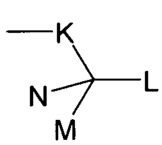

I*

dans laquelle

K représente une liaison simple, alkylène ayant 1 à 9, de préférence ayant 1 à 5 atomes de C, alkénylène ou alkynylène ayant 2 à 9, de préférence ayant 2 à 5 atomes de C, où un, deux ou plus de deux des groupes $-CH_2-$ présents dans tous les trois types de groupe peuvent chacun être remplacés par -O-, -C=O- ou -S-, mais où pas deux atomes de O sont liés directement à un autre et tous les trois types de groupe peuvent optionnellement être substitués par halogène, et

L, M et N chacun, indépendamment les uns des autres, mais différemment les uns des autres et du reste de la molécule incluant le groupe K, représentent hydrogène, halogène, de préférence F, aryle ou cycloalkyle, alkyle ou alcoxy ayant 1 à 11 atomes de C, alkényle, alkényloxy, alkynyle ou alkynyloxy ayant 2 à 11 atomes de C, où un, deux ou plus de deux des groupes $-CH_2-$ présents dans tous les six types de groupe peuvent chacun être remplacés par -O-, -C=O- ou -S-, mais où pas deux atomes de O sont liés directement à un autre et tous les six types de groupe peuvent optionnellement être substitués par halogène,

$Z^1$, si il est présent plus d'une fois, dans chaque cas, indépendamment les uns des autres, représente $-CH_2-CH_2-$, -CH=CH-, -C≡C-, -COO-, -OCO-, $-CH_2O-$, $-OCH_2-$, $-CF_2O-$, $-OCF_2-$, $-(CH_2)_4-$, -CF=CF-, -CH=CF-, -CF=CH-, $-CH_2-$, $-CF_2-$, -CHF-, -O-, -S- ou une liaison simple,

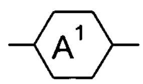,

si il est présent plus d'une fois, dans chaque cas, indépendamment les uns des autres, représente
(a) un radical trans-1,4-cyclohexylène, dans lequel, en addition, un ou plusieurs groupes $CH_2$ non adjacents peuvent être remplacés par -O- et/ou -S-,
(b) un radical 1,4-cyclohexénylene,
(c) un radical 1,4-phénylène, dans lequel, en addition, un ou deux groupes CH peuvent être remplacés par N, ou
(d) un radical choisi parmi le groupe
1,4-bicyclo[2.2.2]octylène,
pipéridine-1,4-diyle, naphtalène-2,6-diyle,
décahydronaphtalène-2,6-diyle et
1,2,3,4-tétrahydronaphtalène-2,6-diyle,

où ces radicaux (a) à (d) et le cycle benzène phénolique peuvent optionnellement être mono- ou polysubstitués par des atomes de F, et

$n^1$ représente 0, 1, 2 ou 3,

**caractérisé en ce qu'**il induit une phase cholestérique dans un cristal liquide nématique et simultanément agit en tant que stabilisateur.

2. Composé selon la revendication 1, de la formule Ia

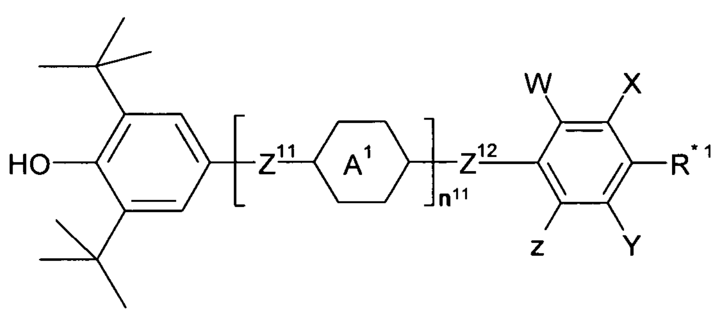

Ia

dans laquelle

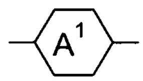

et $R^{*1}$ ont la signification donnée dans la revendication 1, et
$Z^{11}$ et $Z^{12}$ chacun, indépendamment l'un de l'autre, ont la signification donnée pour $Z^1$ dans la revendication 1,

et

$n^{11}$ représente 0, 1 ou 2, et
W, X, Y et Z chacun, indépendamment les uns des autres, représentent H, F, Cl, alkyle ou alcoxy, de préférence ayant 1 à 7 atomes de C.

3. Composé selon l'une des revendications 1 et 2, **caractérisé en ce que**

$R^{*1}$ représente un radical chiral de la formule I*

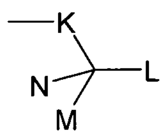

I*

dans laquelle

K représente une liaison simple, alkylène ayant 1 à 5 atomes de C, alkénylène ou alkynylène ayant 2 à 5 atomes de C, où un, deux ou plus de deux des groupes $-CH_2-$ présents dans tous les trois types de groupe peuvent chacun être remplacés par -O-, -C=O- ou -S-, mais où pas deux atomes de O sont liés directement à un autre et tous les trois types de groupe peuvent optionnellement être substitués par fluor, et
L, M et N chacun, indépendamment les uns des autres, mais différemment les uns des autres et du reste de la molécule incluant le groupe K, représentent hydrogène, F, aryle ou cycloalkyle, alkyle ou alcoxy ayant 1 à 7 atomes de C, alkényle, alkényloxy, alkynyle ou alkynyloxy ayant 2 à 7 atomes de C, où un, deux ou plus de deux des groupes $-CH_2-$ présents dans tous les six types de groupe peuvent chacun être remplacés par -O-, -C=O- ou -S-, mais où pas deux atomes de O sont liés directement à un autre et tous les six types de groupe peuvent optionnellement être substitués par fluor,

4. Composé selon au moins une des revendications 1 à 3, **caractérisé en ce que**
$R^{*1}$ représente un radical chiral choisi parmi le groupe des radicaux des formules

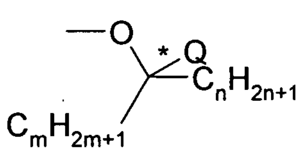

,

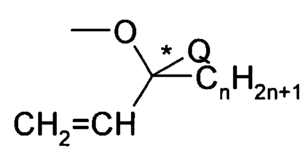

,

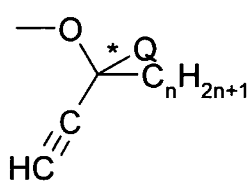

,

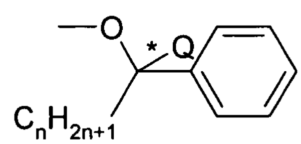

,

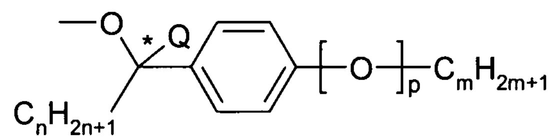

,

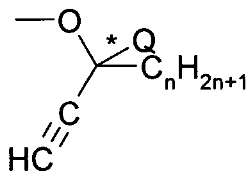

,

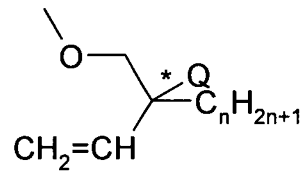

,

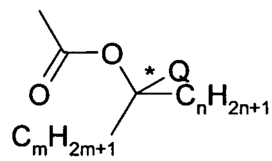

et

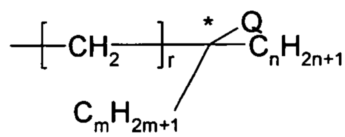

dans lesquelles

Q représente H ou halogène, de préférence H ou F, de préférence H,
n et m sont différents l'un de l'autre et par ailleurs, indépendamment l'un de l'autre, représentent 1 à 11,
p représente 0 ou 1, et
r représente 0 à 4.

5. Composé selon la revendication 4, **caractérisé en ce que**

r représente 0 à 2.

6. Composé selon au moins l'une des revendications 1 à 5, choisi parmi le groupe des composés des formules Ia-1 à Ia-9

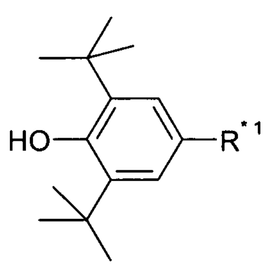

Ia-1

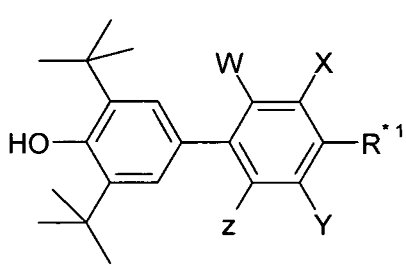

Ia-2

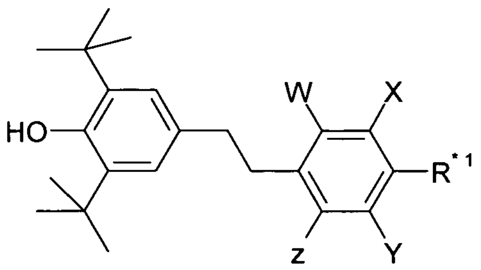

Ia-3

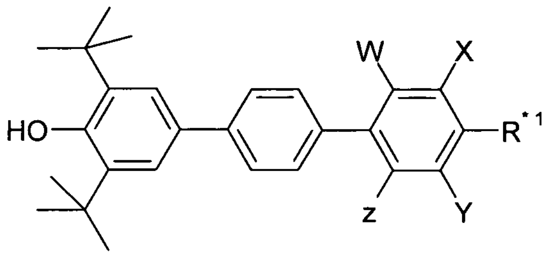

Ia-4

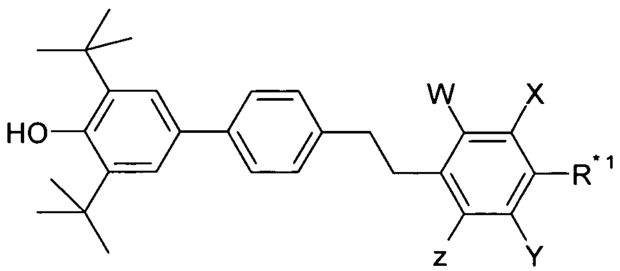

Ia-5

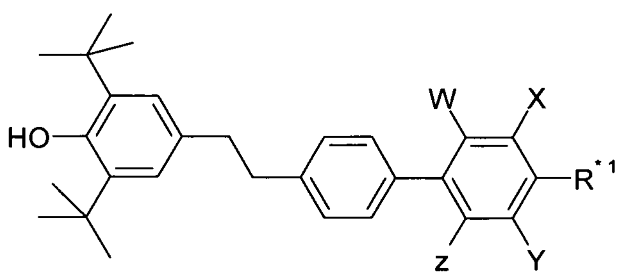

Ia-6

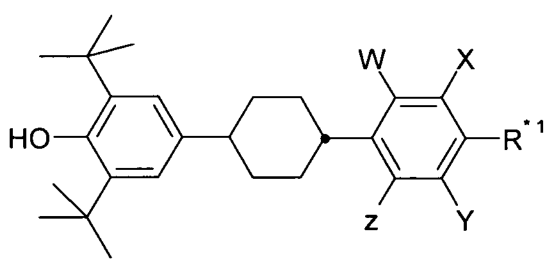

Ia-7

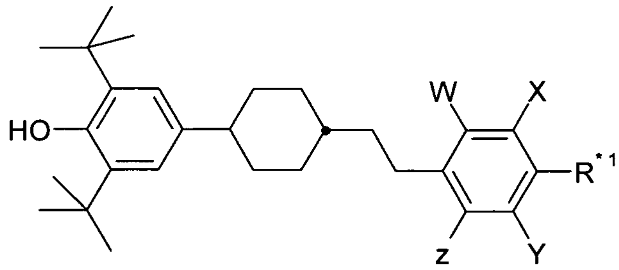

Ia-8

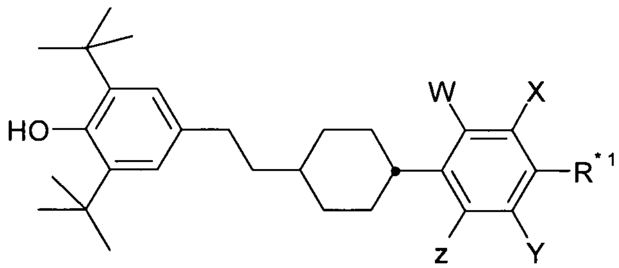

Ia-9

dans lesquelles les paramètres ont la signification donnée dans la revendication 2.

**7.** Composé selon la revendication 6, **caractérisé en ce que** W et Z représentent H.

**8.** Utilisation d'un ou plusieurs composés selon au moins l'une des revendications 1 à 7 dans un mélange de cristal liquide, en tant que stabilisateur ou en tant que dopant chiral et simultanément en tant que stabilisateur.

**9.** Milieu de cristal liquide, **caractérisé en ce qu'**il comprend un ou plusieurs composés selon au moins l'une des revendications 1 à 7.

**10.** Utilisation d'un milieu de cristal liquide selon la revendication 9 dans un affichage électro-optique.

**11.** Affichage électro-optique contenant un milieu de cristal liquide selon la revendication 9.

**12.** Procédé pour la préparation d'un mélange de cristal liquide, **caractérisé en ce qu'**un composé qui induit une phase cholestérique et simultanément agit en tant que stabilisateur est ajouté au mélange de cristal liquide.

**13.** Mélange de cristal liquide, **caractérisé en ce qu'**il a été préparé par le procédé selon la revendication 12.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19539141
- DE 10117224
- EP 0217239 A


### In der Beschreibung aufgeführte Nicht-Patentliteratur


- **Mäurer, M.** *Chem. Ber.,* 1992, vol. 125 (4), 857-865